(19) Europäisches Patentamt
European Patent Office
Office européen des brevets

(11) Publication number: 0 255 026 A2

# (12) EUROPEAN PATENT APPLICATION

(21) Application number: 87110579.7

(22) Date of filing: 22.07.87

(51) Int. Cl.³: G 01 N 35/00
C 12 M 1/00, G 01 N 33/48

(30) Priority: 23.07.86 JP 174515/86
23.07.86 JP 174516/86
23.07.86 JP 174517/86
23.07.86 JP 174518/86

(43) Date of publication of application:
03.02.88 Bulletin 88/5

(84) Designated Contracting States:
AT BE CH DE ES FR GB GR IT LI LU NL SE

(71) Applicant: Takeda Chemical Industries, Ltd.
27, Doshomachi 2-chome Higashi-ku
Osaka-shi Osaka, 541(JP)

(72) Inventor: Amano, Toshio
3-20, Ankojicho 4-chome
Takatsuki Osaka 569(JP)

(72) Inventor: Imada, Isuke
18-10, Tsuruyamadai 4-chome
Izumi Osaka 594(JP)

(74) Representative: Eisenführ & Speiser
Martinistrasse 24
D-2800 Bremen 1(DE)

## (54) Automatic analysis method and apparatus for enzyme reaction.

(57) An automatic analysis method of and apparatus for the full automation from the pretreatment of the reaction analysis to the data processing in the enzyme reaction automatic analysis system, wherein the operation of the pretreatment step of many samples are adapted to be sequentially performed with full automation by the use of robots, computers to improve the saving, efficiency and measurement accuracy. Also, the pretreatment steps of weighing the samples in many sample tubes, adding the given amount of solvent corresponding to the weighing value and placing the samples into the dissolution vessel to dissolve the sample in the solvent is adapted to be sequentially performed with full automation with the use of the robot, computer and electronic balance, efficiency and measurement accuracy. Furthermore, the filtration, concentration and the injecting operations into the HPLC are automatically automated, the automatic apparatuses are coupled to each other so that the filtration, concentration, injecting operations are adapted to be sequentially automated with full automation on the on-line to improve the saving, efficiency and accuracy.

Fig. 7

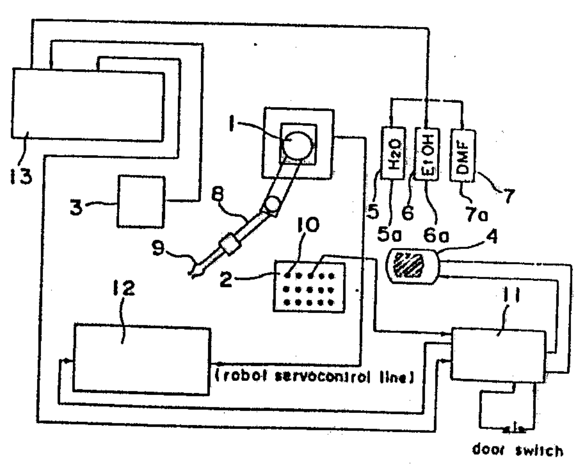


EP 0 255 026 A2

BACKGROUND OF THE INVENTION

The present invention relates to an automatic analysis method for enzyme reaction and an apparatus thereof, and, more particularly, to a method and an apparatus for the full automation from the pretreatment to the data processing of the reaction analysis in the enzyme-reaction automatic analysis system, where the assay of the effects of substances on the lipoxygenase and cyclooxygenase systems in the arachidonic acid cascade is performed with respect to various compounds with the minimum necessary labor through the measurement automation to provide the data for the selection of the secondary screening compound.

In the biochemistry field, it is extremely important to analyze the effects of various materials on the enzyme reaction for the primary screening of a biologically -active substance and the elucidation of the action mechanism thereof. Particularly, to find out an active substance, it is required to analyze the effects of many substances on the constant enzyme reaction.

Generally, the automatic analytical system (what is called "Laboauto") is divided into the pretreatment of the sample, analysis and data processing/analysis. Conventionally, the laboratory automation indicates the automatic analysis and the data processing/analysis, and as the pretreatment is complicated and contains various methods, it is not often introduced.

Thus, the operations required to be carried out at the pretreatment step, for example, weighing, dissolution, dilution, reaction, filtration, concentration and injection into the analytical apparatus of many samples are performed by the manual method, and, as these operations are complicated, there cause lots of problems such that more time is required, the efficiency is deteriorated, and the quantitative property is lowered, thus resulting in poor measurement accuracy.

SUMMARY OF THE INVENTION

Accordingly, the all operations of the pretreatment step for many samples are adapted to be sequentially performed with full automation by the use of robots, computers and so on to improve the saving, efficiency and measurement accuracy.

The present invention is to provide an apparatus for performing, the elucidation of the physiological and pharmacological roles of the useful, minor-tissue components such as arachidonic acid metabolites, the influences upon pathologic physiology or for screening substances regulating these metabolism, and more particularly, a method of and an apparatus for observing and controlling the weighing, dissolution, dilution, pipetting process by a robot operation, and incubator, preservation vessel, filtration, concentration, injection, high performance liquid chromatograph (HPLC) and the automatic process of a data processing apparatus.

Also, the operation of the pretreatment steps, weighing many samples, adding the given amount of solvent corresponding to the weighing value and placing the sample in the solvent into the dissolution vessel to dissolve the sample is adapted to be sequentially performed with full automation with the use of a robot, a computer and an electronic balance, thus resulting in improved saving, efficiency and measurement accuracy.

In addition, according to the present invention, the pretreatment is automated with the use of the robot system, the sample dilution, sampling into a reaction tube and pipetting of the reaction mixture are also automated, and the whole analysis system including the pretreatment step is fully automated to improve the saving, efficiency and quantitative property.

Furthermore, the filtration, concentration and injecting operations into the HPLC are automated, the automatic apparatuses are coupled to each other so that the filtration, concentration, injecting operations are adapted to be sequentially automated with full automation on the on-line to improve the saving, efficiency and accuracy.

To achieve the above-described object, the present invention provides a method and an apparatus for performing sequentially automatically the weighing operation of a sample, the adding operation of solvent to the sample, the dissolving operation of the sample in the solvent, the adding operation of the solvent to the sample solution, the pipetting operation of the diluted sample solution into a reaction tube, the adding operation of enzymes into the reaction tube, the moving operation of the reaction tube onto a preservation vessel, the filtering operation of the reaction mixture in the reaction tube to get a filtrate, the concentrating operation of the filtrate, and the pipetting operation into an analysis apparatus.

In the above-described method for automatically carrying out all the steps of grasping a sample tube retained in a rack with a chuck provided on a movable arm of an automatic robot for weighing of the sample, adding solvent, dissolution so as to move it to an electronic balance, a solvent dispenser, a dissolution vessel, adding of the solvent predetermined in accordance with the weighing value of the sample after the weighing operation by the electronic balance, placing the sampling tube into the dissolution vessel to dissolve the sample after the addition, moving the sample tube with the dissolution of the sample in it onto the rack for dilution provided in a reaction apparatus of the next step; grasping each nozzle of a dilution dispenser, a sampling pipetter and an enzyme reaction mixture dispenser with the chuck of the automatic robot to move it into the sample tube retained in the rack for dilution use and the reaction tube retained in the rack placed in the reaction vessel for the addition of diluted solution, injection into the reaction tube, addition of enzyme reaction mixture so as to perform the injecting operation, adding the diluted solution from the dilution dispenser into the sample tube located in the dilution rack to perform the diluting operation into given times, sampling the diluted solution of the sample tube from the sampling pipetter to inject it into the reaction tube, sequentially injecting enzymes and factors necessary for enzyme reaction into the reaction tube at intervals of a given time, grasping the reaction tube with the robot to move it onto the preservation vessel after the reaction; grasping the reaction tube retained in the preservation vessel with the robot to place it in the position of a reaction mixture sampling needle disposed in filtration/concentration/analysis apparatus, filtering the reaction mixture through a filtration unit after the sampling operation of reaction mixture with the sampling needle, feeding eluate into a column for concentration use after the adsorption of the filtrate onto the column for the concentration use to inject it into an analysis column for the analysis by a HPLC so that the obtained data is processed by a processing apparatus; and automatically controlling the operations of the robot in accordance with a program input into a computer to automatically perform all the steps from weighing to analysis.

Also, in the above-described enzyme reaction automatic analysis apparatus, the automatic weighing and dissolving apparatus comprises a rack for retaining many sample tubes on the different periphery of the robot to be automatically controlled, an electronic balance, a solvent dispenser, a dissolution vessel with an ultrasonicator thereon so that the sample retained in the rack is grasped with a chuck disposed on movable arm of the robot to move it onto the electronic balance, solvent adding apparatus, dissolution vessel; the dilution reaction apparatus of the dissolved sample comprises a rack for dilution use for retaining many sample tubes in the different position on the periphery of the robot to be automatically controlled, a incubator provided with the rack for retaining many reaction tubes, a dilution dispenser, a sampling pipetter, a plurality of dispensers for injecting enzymes, a preservation vessel provided with a rack for retaining the reaction tube, so that the nozzle of each injector is grasped with the chuck of the robot to move it onto the sample tube and reaction tube to perform the injection, the reaction tube of the incubator is grasped with the chuck of the robot to move it onto the rack of the preservation vessel; and the filtration/concentration/injection apparatus (FCI) of reaction solution comprises a sampling needle for sampling the reaction mixture from the reaction tube through the operation by a syringe pump, a filtration unit of a filter paper continuous winding type for filtering the sampling reaction mixture, a concentration column for concentrating the filtrate, a valve for feeding the eluate into the concentration column to inject it into the analysis column, so that each of these apparatuses is so disposed that the filtration, concentration, injection may be performed on the on-line.

Although any enzyme may be used in the present invention, something showing biological activity is preferable with the substrate of the enzyme reaction or the product being extremely small in amount. For example, the lipoxygenase or the cyclooxygenase is preferable with the products being leukotrienes, prostaglandins and thromboxanes. As the enzyme preparation, tissue cells or cell lines can be used in addition to purified enzymes. For example, soy bean lipoxygenase refined from the soy bean, rat basophilic leukemia cell (RBL-1), porcine or rat platelets are used.

As described hereinabove, the enzyme reaction starts through the addition of substrate and factors necessary for the enzyme reaction under the existence of sample solution or under the existence of solvent only, while the enzyme reaction is stopped through the addition of organic solvent or through the reduction in the temperature. The analysis of the enzyme reaction mixture is performed by the HPLC. The enzyme reaction mixture is filtered, concentrated by a constant amount, and the obtained concentrated solution is injected into the HPLC apparatus for analysis. The results of the analysis show an inhibition or acceleration of the enzyme reaction as compared with a case where only the solvent of the sample has been added.

Also, to achieve the above-described object, the present invention provides an automatic weighing and dissolution apparatus for samples, wherein a rack for retaining a plurality of sample tubes for free taking-out operations in the different positions on the periphery of the automatic robot, an electronic balance, a solvent adding apparatus, a dissolution vessel with an ultrasonicator on it, so that the sample tube in the rack into which the sample is thrown is freely grasped with a chuck disposed on a movable arm of an automatic robot to sequentially move it automatically onto the electronic balance, solvent adding apparatus and dissolution vessel, and a computer for controlling the operation of the robot, setting the solvent adding amount calculated in accordance with the weight of the sample weighed by the electronic balance, adding the solvent into the sample tube, for automatically controlling each of the apparatuses such as dissolution vessels into which the sample tube after the addition of the solvent is placed.

Furthermore, in order to achieve the above described object, the present invention provides an automatic dilution reacting apparatus which comprises a rack for retaining a plurality of sample tubes, a dilution dispenser

a sampling pipetter, an incubator having a plurality of reaction tubes disposed in it, and a reaction mixture dispenser, a nozzle washing vessel disposed on the positions different on the periphery of an automatic robot, nozzles mounted respectively through flexible pipes on each of the dispensers, these nozzles being detachably placed on a nozzle stand provided in the operation range of the robot, while each of the nozzles is freely grasped with a chuck provided on a movable arm of the robot so that it may move into the opening of the sample tube in the rack or into the opening of a reaction tube in the incubator; and wherein the robot is operated by a controller to inject the solvent into the sample tube in the rack from the dilution dispenser, the dilution sample is pipetted from the sample tube by the sampling pipetter to be injected into the reaction tube within the incubator, the reaction mixture such as cells as enzyme solution, arachidonic acid, ethanol are sequentially added at intervals of given time from the reaction mixture dispenser into the diluted sample located within the incubator for the reacting operation.

Furthermore, to achieve the above-described object, the present invention uses a filtration system for on-line use, instead of the conventional centrifugal separation system, to remove the admixture such protein or the like. A system for continuously winding the filter paper which is simpler, more inexpensive in apparatus among the filtration system, i.e., a system of having band-shaped filter paper placed on the passage, taking it up after the filtration to replace it with the new paper is adopted. Also, in the concentration, the present system adopts the concentration method by a precolumn, because the reversed phase HPLC is used in the identification, estimation of the sample. Namely, a preliminary column (concentration column) small size for the sample adsorption use is disposed before an analysis column, and the sample is dissolved in solvent which is higher polarity as compared with the eluate. When it is moved through the column, the sample is adsorbed. When it is continuously guided into the analysis column from the concentration column with the eluate, the concentration may be performed by the on-line at the same time with the separation/estimation. The sample of 1 mℓ is injected into the concentration column, so that the sensibility increase of 20 through 50 times is caused in the injection amount (20 through 50 μℓ) to the conventional HPLC. Also, the filtration apparatus is coupled to the concentration apparatus, furthermore an HPLC injecting apparatus with a six-way valve switch is mounted to become FCI. A series of operation for filtration → concentration → injection to the HPLC may be automatically effected as the apparatus.

Concretely, the present invention provides the FCI of the sample solution, which is provided with a sampling needle for sampling the sample solution from the sample tube, a syringe pump for operating the sampling needle, a filtration unit for filtering the sampled sample solution by the filter-paper continuous winding type, a six-way valve with a sampling loop on it to which the filtered sample solution, a six-way valve with a concentration column on it, a water pump which feeds the sample solution in the loop of the six-way valve with a sampling loop together with water into the concentration column of the six-way valve with the concentration column.

BRIEF DESCRIPTION OF THE DRAWINGS

These and other objects and features of the present invention will become apparent from the following description taken in conjunction with the preferred embodiment thereof with reference to the accompanying drawings, in which:

Fig. 1 is a schematic plane view of an automatic weighing dissolution apparatus;

Fig. 2 is a schematic plane view of an automatic dilution reacting apparatus;

Fig. 3 shows a view of an automatic filtration, concentration, injection apparatus, (A) being a front face view, (B) being a plane view thereof;

Fig. 4 is an enlarged view of a chuck of a robot, (A) being a front view, (B) being a plane view thereof;

Fig. 5 shows a view of the rack, nozzle of Fig. 1 and a dissolution vessel, (A) being a plane view, (B) being a front view thereof;

Fig. 6 shows a view of a sample holder of an electronic balance, (A) being a front view, (B) being a side view, (C) being a plane view thereof;

Fig. 7 is a control circuit diagram of the apparatus of Fig. 1;

Fig. 8 is a schematic construction view of an interface unit;

Fig. 9 is a block diagram showing the operation sequence of the apparatus of Fig. 1;

Fig. 10 is a flow sheet showing the operation sequence;

Fig. 11 is an enlarged view of a rack 21 of Fig. 2, (A) being a plane view, (B) being a front view thereof;

Fig. 12(A) is a front view of a nozzle stand of a dilution dispenser and a sampling pipetter;

Fig. 12(B) is a front view of a nozzle stand of a reaction mixture dispenser;

Fig. 13 is an enlarged view of a incubator, (A) being a plane view, (B) being a front view thereof;

Fig. 14 is an enlarged view of a preservation vessel, (A) being a plane view, (B) being a front view thereof;

Fig. 15 is a construction view of the system of an automatic dilution reacting apparatus;

Fig. 16 is a view showing electric connections;

Fig. 17 is a flow chart showing the operation sequence;

Fig. 18 shows the arrangement of sample tubes of the rack 21;

Fig. 19 is a principle view of filtration, concentration, injection apparatuses shown in Fig. 3;

Fig. 20 is a front view of an operation panel;

Fig. 21 is a block view showing the operation sequence of the filtration, concentration, injection apparatuses;

Fig. 22 is an operation circuit diagram of each apparatus;

Fig. 23 (a), (b), (c) and Fig. 24 show chromatogram of various kinds of sample solution;

Fig. 25 shows a chromatogram operated by this apparatus;

Fig. 26 shows a chromatogram in the conventional method;

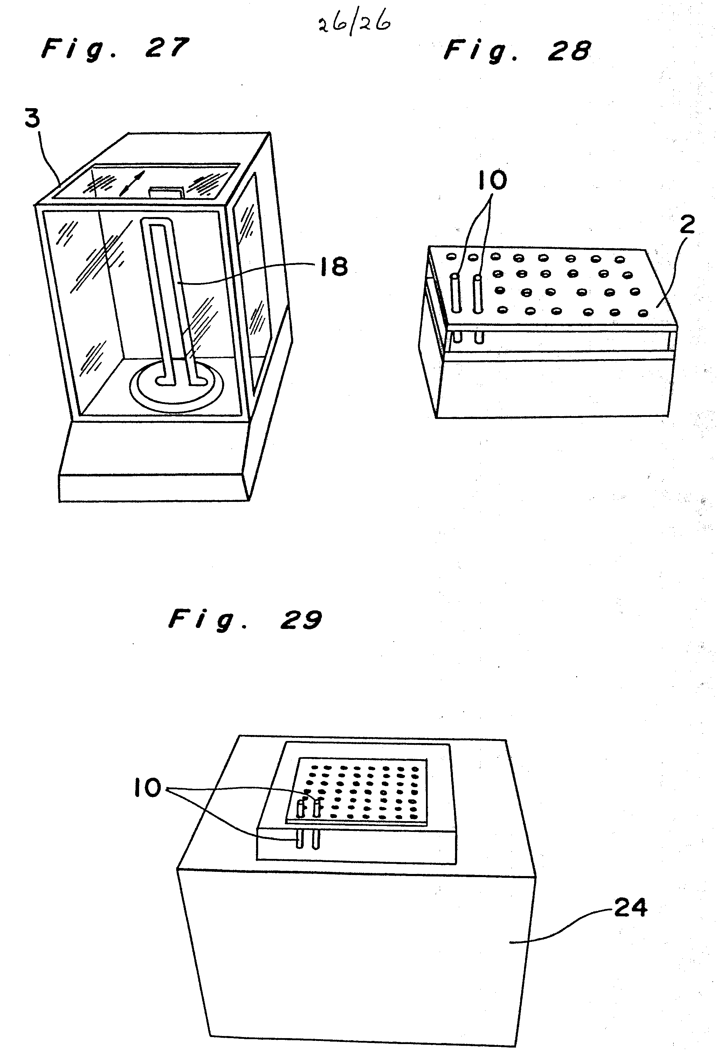

Fig. 27 is a perspective view of the electronic balance;

Fig. 28 is a perspective view of the rack; and

Fig. 29 is a perspective view of the incubator.

DETAILED DESCRIPTION OF THE INVENTION

Before the description of the present invention proceeds, it is to be noted that like parts are designated by like reference numerals throughout the accompanying drawings.

The present automatic analysis apparatus is composed of an automatic weighing/dissolution apparatus shown in Fig. 1, an automatic dilution reacting apparatus shown in Fig. 2, an FCI and data processing apparatus (not shown).

First, the construction and operation of the automatic weighing, dissolution apparatus shown in Fig. 1 will be described.

The present automatic weighing, dissolution apparatus is adapted to sequentially perform by the use of a robot, a computer, an electronic balance or the like, the actions of the pretreatment steps such as weighing operation of samples thrown into many sample tubes, adding operation of a given amount of solvent corresponding to the weighing value, and placing operation of the samples into a dissolution vessel for dissolving the samples into solvent.

As shown in Fig. 1 and Fig. 4, an automatic robot 1 is disposed in the approximately central portion of the work station S1 with a rack 2 for retaining a sample tube, an electronic balance 3, a dissolution vessel 4 with a ultrasonicator attached thereto, solvent adding apparatuses (constant-amount dispenser) 5, 6, 7 being disposed in different positions within the operation range around the robot 1. The sample tube 10 is held with a chuck 9, which is disposed on the tip end of an arm 8 of the robot 1 so that it may be carried to each of the apparatuses. It is noted that the dissolution vessel 4 is constructed as a dissolver, an extractor of mixer type or vibration type and the like.

Also, the work station S1 has an interface unit 11, a robot controller 12, a factory computer 13 disposed beyond the operation range of the robot 1. The computer 13 is composed of a CPU (central processing unit) 14, a keyboard 15, a CRT (display apparatus) 16 and a floppy-disc (memory apparatus) 17.

Although a cylindrical coordinate, a polar coordinate, a rectangular coordinate or a multi-joint type, etc. are used as the robot 1, the multi-joint robot of a horizontal type is used in the present embodiment. The robot 1 is 305 mm + 295 mm in arm length, 220° for one shaft in the operation range, 300° for two shafts, 150 mm for Z, 360° for W in operation range, 3200 mm per second in maximum composite speed. The present robot 1 has advantages in the speed is faster, action range is wider, accuracy is better, the operation is easier to assemble for multiple objects. The robot 1 is operated by the robot controller 12 to move the arm 8 into the given position to open, close the chuck 9 in the position. As shown in Fig. 4, the chuck 9 detachably grasps the upper side portion of the sample tube 10 by a finger 9a mounted on the chuck 9 to carry the sample tube

10. It is to be noted that the finger 9a is made of polyurethane so that impact may not be applied upon the sample tube 10 when the sample tube 10 has been grasped.

The specification of the robot controller 12 for operating the robot 1 adopts the maximum simultaneous four shafts in the number of the control shafts, a PTP system in route control system, a semi-closed loop system by the rotary encoder in control system, the number of position settings is 500 in total of 30 steps x 15, 50 steps x 1, the speed is variable in nine stages with 10 in input, 10 in output (0.01 through 60.00 seconds) in timer 8, 10 in memory.

As shown in Fig. 5, the rack 2 has a sample tube 10 inserted into a hole for sample tube erection use, many sample tubes 10 retained in longitudinal/lateral parallel. For easier insertion of the sample into the tube 10, it is adopted to be removed from the work station S1 with one touch.

The electronic balance 3 which is 30 g in weighing limit, 0.01 mg in weighing accuracy is capable of weighing 1 through 3 mg. A sample holder 18, shown in Fig. 6, of the balance 3 is made of aluminum as light as possible and is set to the weight of 22 g or lower. The robot 1 is adopted to be smoothly performed so that the robot 1 may take in and out the sample tube 10 from above the electronic balance 3.

The door of the electronic balance is adapted to open or close by a two-way cylinder (not shown) to be actuated through an electromagnetic valve which is connected with the robot so as to be controlled together by a controller of the robot. Also, when the robot 1 places the sample tube 10 on the upper portion of the holder 18 with a spring 19 being mounted on the sample receiver, the shock with respect to the balance is adopted to be reduced so that the balance may be safely used. Also, the door of the electronic balance 3 is adopted to be closed and opened by the finger 9a of the chuck 9 of the robot 1.

A heater with a temperature controller 4a is mounted on the dissolution vessel 4 with the ultrasonicator on it. The heater is turned on, off in control by the computer 13 to retain the temperature in the vessel at an optional constant temperature such as usually at 37°C. Also, when the sample tube 10 is placed by the robot 1 into the dissolution vessel 4, the ultrasonicator turns on. When the sample tube 10 is taken out, it is adapted to stop. The ultrasonicator is continuously used to cause the heat to prevent the temperature in the vessel from being increased.

As shown in Fig. 5, the solvent adding apparatuses 5, 6, and 7 are adapted to add the solvent of different kinds to the sample tube 10 respectively from the nozzles 5a, 6a, and 7a. A digital diluter/pipetter is used as the solvent adding apparatus. A plurality of solvents are set, because some samples are not dissolved only in one type of solvent. In the present embodiment, water is added by the apparatus 5, ethanol by the apparatus 6, dimethyl formamide by the apparatus 7.

The addition amount by the solvent adding apparatuses 5 through 7 is set through the calculation from the molecular weight and the concentration which are inputted in advance by the computer 13 in accordance with the weight of the sample weighed by the electronic balance 3, so that the set amount is added into the sample tube from the nozzle.

At the completion of the solvent addition, the sample tube 10 is moved a little by the robot 1 to cause the solvent liquid at the nozzle tip end to come into contact against the inner wall of the sample tube 10 so that the given amount of solvent may be completely injected into the sample tube 10.

The entire controlling line of this apparatus is shown in the construction view of Fig. 7. The schematic construction of the interface unit 11 is shown in Fig. 8. The program input into the computer 13 is shown in the block diagram of Fig. 9. The sample information, each as the total number of the samples, the number of the sample, the molecular weight, the dissolution concentration and the solvent name are kept input into the computer 13 in advance at the start time of the program, so that the optional dissolution may be performed.

The weighing operation and the dissolving operation by the apparatus will be made in the following order described hereinafter. As the operation is effected in such an order as in Fig. 10, the arm 8 of the robot 1 is restored to the original point, before the start of the program, and the empty tubes 10 are arranged in the rack 2.

(1) The sample information is input into the computer 13.

Namely, the total number of the samples, the lot number, the molecular weight, the dissolution concentration, and the using solvent are input.

(2) The weighing of the empty sample tube 10 is effected.

Namely, the door of the electronic balance 3 is opened with the chuck 9 of the robot 1 (it is to be noted that all the following operations are performed by the finger 9a of the chuck 9 of the robot 1). → The sample tube 10 is moved from the rack 2 into the balance 3 and is on the holder 18. → The door is closed. The sample tube 10 is weighed (tare weighing). → The door is opened. → The sample tube 10 is moved into the rack 2 from the balance 3. → The above-described operation is repeated for all the sample tubes 10.

(3) Then, the sample is manually added by 1 through 3 mg to each of the sample tube.

(4) Then, the sample weighing operation is effected.

As in the weighing operation of the tare, the sample 10 is moved into the electronic balance 3 from the rack 2 with the chuck 9 of the robot 1 for the weighing operation. It is restored into the rack 2 after the weighing operation. The operation is repeated for all the sample tubes.

The weighing data by the balance 3 is input into the computer 13. The kind of the solvent to be added, and the amount of addition are set through the calculation from the molecular weight and the concentration which are inputted in advance into the computer 13.

(5) Then, the solvent addition is performed.

Namely, the sample tube 10 is grasped from the rack 2 by the finger 9a of the chuck 9 of the robot 1, is moved to the nozzle tip end of either specified solvent adding apparatuses 5, 6, and 7 to add the solvent of the set amount for the calculated time. After the injection, the arm 8 of the robot 1 is moved a little to cause the solvent of the nozzle tip end to come into contact against the inner wall of the sample tube 10 to completely add the set amount.

When the solvent of two kinds or of three kinds is required, the sample tube is moved to the other adding apparatus after the addition by one adding apparatus and is added. The sample tube is returned to the rack 2 after the solvent addition. The operation is repeated for all the sample tubes.

(6) Then, the dissolution of the sample is effected.

Namely, the sample tube 10 in the rack 2 is moved into the dissolution vessel 4 with an ultrasonicator by the robot 1 to dip one portion of the sample tube 10. The sample is kept for thirty seconds within the dissolution vessel 4 and is in the solvent. The dissolution vessel 4 is normally maintained at 37°C. When the sample tube 10 is placed into vessel 4, the ultrasonicator turns on. When the sample tube is taken out, the ultrasonicator is turned off. The sample tube 10 which has been taken out of the dissolution vessel 4 is applied upon the filter paper to remove the attached water and is returned to the original place of the rack 2 by the robot 1. This operation is performed all the sample tubes 10 to dissolve all the samples.

As shown in the O model frame of Fig. 10, each operation is displayed by the CRT 16. Also, such test results of the sample tube 10 as shown hereinabove are printed out on a given piece of paper by a printer (not shown) so that the test results may be easily confirmed.

The enzyme dilution reacting apparatus shown in Fig. 2 has in the different positions around the automatic robot a rack for retaining a plurality of sample tubes, a dilution dispenser, a sampling pipetter, a incubator with a plurality of reaction tubes disposed therein and a reaction mixture dispenser. The nozzles are mounted respectively through flexible pipes in each dispenser. These nozzles are detachably in the nozzle stand disposed in the operation range of the robot. Each of the nozzles are freely grasped with the chuck provided on the arm of the robot so that it is adapted to be moved into the opening of the sample tube in the rack or of the reaction tube in the incubator. The robot is operated by the controller to inject the diluted solution into the sample tube in the rack from the dilution dispenser, the diluted sample is sampled from the sample tube by the sampling pipetter to inject it into the reaction tube within the incubator. The reaction mixture which is enzyme solution such as cell, arachidonic acid, ethanol, etc. is sequentially added from the reaction mixture dispenser into the diluted sample in the incubator after the given time so as to the reacting operation.

As shown in Fig. 2, an automatic robot 20 is disposed in the central position on the rear side of the work station S2, a rack 21 for diluting the original solution, a dilution dispenser 22, a sampling pipetter 23, a incubator 24, reaction mixture dispensers 25A, 25B, 25C and a preservation vessel 26 are disposed clockwise forwardly from the left on the periphery different within the operation range of the robot 20. Also, nozzle stands 27, 28, 29, 30, and 31 are set respectively on the side of each of the dispensers 22, 23, 25A, 25B, and 25C to detachably retain nozzles 32a through 36a which are integrally formed on the tip ends of the flexible pipes connected with the respective dispensers 22 through 25C. The nozzle holders 32b through 36b are engaged with these nozzles 32a through 36a. The nozzle holders 32b through 36b are grasped with a chuck 20b disposed on the tip end of an arm 20a of the robot 20 so that they are adapted to be moved into the opening portion of a sample tube 37 in the rack 21 or of a reaction tube 38 in the incubator 24. Also, a robot controller 39 is disposed beyond the operation range of the robot 20 on the work station S2 to automatically control the robot 20 by the robot controller 39.

The cylindrical coordinate, polar coordinate, rectangular coordinate and multi-joint, etc. are used as the robot 20 as in the automatic weighing/dissolution apparatus, but in the present embodiment, the horizontal multi-joint type robot 20 is used. The robot 20 is cylindrical in the work range of 800 mm in radius, 300 mm in height. The first articulation and the second articulation (X,Y axes), the third articulation (Z axis), the fourth articulation ($\theta$ axis) may be simultaneously controlled, the portable weight is 10 kg with 75/100 in speed, the reproducibility of the position is ± 0.05 mm.

The present robot 20 has advantages in that the speed is faster, the operation range is wider, the precision is better, the operations are likely to be built-up for multiple objects. The robot 20 is operated by the robot controller 39 to move the arm 20a to the given position so as to perform the opening, closing operations of the chuck 20b. The chuck 20b detachably grasps the nozzle holders 32b through 36b by the finger mounted on the chuck 20b as in the robot 1 or freely grabs the reaction tube 38 to carry it. Also, the object grasped and carried with the chuck 20b with the finger being 24 mm in full opening is restricted in weight to 12 mm in diameter, and the sample tube 37, the reaction tube 38, and the nozzle holders 32b through 36b are the same in diameter. It is to be noted that the finger is similar to 9a of Fig. 4.

The rack 21 for diluting the original solution is of such construction as shown in Fig. 11. The sample tube stand has the holes of 12 mm in diameter arranged at the pitch of 25 mm in eight columns, eight lines in accordance with the instructions of the robot 20. The racks in the incubator 24 and the preservation vessel 26 are similar in construction. Some sample tubes 37 to be retained by the rack 21 has the sample solution thrown thereinto, other sample tubes are empty.

The dilution dispenser 22 uses a digital diluter/pipetter, has original point detection, self-flushing, self-examination, air layer constituting functions by a two-syringe system through the controlling operation of the 8-bit microprocessor, is 5 through 250 μℓ on the test side, 0.05 through 9.99 mℓ on the sample side in capacity range. When the precision is ± 1% of the syringe maximum capacity, the operation speed is three seconds, in half cycle with syringe of 1 mℓ and also five seconds with syringe of 2.5 mℓ.

The flexible pipe 32 for feeding the diluted solution into the sample tube 37 from the dispenser 22 is Teflon tube (2m in length, 1 mm in inner diameter) made into coil-shaped one of 10 mm in diameter. The nozzle 32a is formed at the tip end of the tube to integrate the flexible pipe with the nozzle. The cylindrical Teflon-made nozzle holder 32b of 12 mm in diameter, 70 mm in height is fixedly engaged with the nozzle 32a in the location of 100 mm from the tip end to prevent the flexibility in the expansion and to retain the injection precision. The sampling pipetter 23, the reaction mixture dispensers 25A, 25B, 25C and the flexible pipes 32 through 36 coupled to them, the nozzles 32a through 36a, the respective nozzle holders 32b through 36b are the same in construction as the dilution dispenser 22.

Flushing vessels 27a, 28a for flushing the nozzles 32a, 33a are provided as shown in Fig. 12 (A) on the nozzle stands 27, 28 for detachably retaining the nozzles 32a, 33a of the dilution dispenser 22 and the sampling pipetter 23, so that the tip end of the nozzle for each completion of the sampling is dipped to flush the exterior side. The flushing operation of the nozzle interior is performed simultaneously with the injection of the diluted solution, so that the flushing operation is not required to be effected for each sample.

On the other hand, the nozzles 34a through 36a of the reaction mixture dispensers 25A, 25B, 25C are not required to be flushed, so that the flushing vessels are not disposed as shown in Fig. 12 (B) on the nozzle stands 29, 30, 31.

The incubator 24 is of such construction as shown in Fig. 13 (A), (B) and has a circulating jet type thermostat 40 at the lower stage. The apparatus 40 circulates thermostatic water of the jet type which has the accuracy of controlling the temperature of 0.1°C by the thermistor ON/OFF system between the room temperature +5°C through 60°C (heater 400W). Also, a magnetic stirrer 41 is provided at the middle stage of the incubator 24, a thermostat 42 is disposed at the upper stage to retain the interior of the thermostat 42 at 37°C in the embodiment. A rack 43 similar to the rack 21 is disposed in the thermostat 42. Also, a blister-sterol made liquid-level detector (not shown) of floating type is mounted in the thermostat 42. When the liquid in the vessel reduces, the blister-sterol lowers to operate the alarm by a microswitch mounted under it to stop the robot 20. The reaction tube 38 to be longitudinally, horizontally in parallel in the rack 43 to be set in the incubator 24 is the same in diameter, i.e., 12 mm as the sample tube 37. A magnetic stirrer bar of 5 mm in diameter is placed in advance in the reaction tube 38 so that the stirring of hand-drum shape is adapted to be caused by the magnetic stirrer 41, which is provided with a magnet of 20 cm in diameter. The stirring for 64 reaction tubes 38 is adapted to be simultaneously effected by one stirrer. Also, a temperature runaway preventing apparatus which may be set to the optional is mounted as the heater is used in the present incubator, and in the present embodiment, the temperature is retained at 37°C.

The preservation vessel 26 is of such construction as shown in Fig. 14 (A), (B). Water is circulated by a cool-water circulating machine, cool pump, of a liquid expansion type having the temperature adjuster with 0°C through the room temperature in temperature range and about 120 Kcal per hour in cooling performance. Also, an excessive heat/excessive cool preventing apparatus which may be set at the optional temperature is mounted as a safety apparatus with the temperature being 0°C in the present embodiment. The cooling water retained at the set temperature is circulated into the cooling vessel to retain the temperature at the optional temperature with a rack 45 similar to the rack 21 being disposed in the preservation vessel 26. Also, as a floating type of liquid face detector (not shown) is mounted in the thermostat 44, the blister-sterol lowers as the liquid in the vessel reduces. The micro-switch mounted under it actuates the alarm to stop the robot 20.

The FCI which performs filtration, concentration of the reaction mixture, shown in Fig. 3, and performs by the on-line the injection to the HPLC is disposed, adjacent to the work station S1 of the automatic dilution reacting apparatus, so that the reaction tube 38 which is preserved by the preservation vessel 26 is adapted to be moved onto the FCI by the robot 20.

The construction of the system of the automatic dilution reacting apparatus of the present invention is shown in Fig. 15. The robot 20 has the robot controller 39 connected with a sequencer 100. The dispensers 22, 23, 25A, 25B, 25C, the incubator 24, the preservation vessel 26 and the FCI, the HPLC, the door switch for safety measure use, the temperature surveillance apparatus, and the liquid face detector are also connected with the sequencer 100 of which electric connection is shown in Fig. 16. The operation of the present dilution reacting apparatus is shown in the flow chart of Fig. 17. The analytical method has two methods of the primary assay of the rough investigation and the secondary assay of the minute investigation.

The operation by the present apparatus will be described hereinafter when lipoxygenase is used as enzyme solution.

RBL-1 is used as the lipoxygenase, and the cells cultured in BPM-1640 culture solution containing 10% bovine embryo blood serum are washed, thereafter are suspended in the saline. The RBL-1 is prepared as 1 x $10^7$ cell per mℓ. The cell suspension is added from the reaction mixture dispenser 25A and arachidonic acid is adapted to be added from the dispenser 25B, ethanol from the dispenser 25C. Also, dimethyl formamide is adapted to be injected from the dilution dispenser 22.

At the operation start for the following processes, the arm 20a of the robot 20 is restored to the original point by the robot controller 39. Thereafter, the operation of the robot 20 is effected by the robot controller 39. Also, respective dispensers 22 through 25C operate so that reagent filled in respective reagent bottle in advance is full in the cylinder and the flexible pipe.

(1) First, the diluting operation of he sample is performed. Namely, the nozzle holder 32b of the nozzle 32 of the dilution dispenser 22 is grasped with the chuck 20a of the robot 20 to move the nozzle 32 for sampling by the dilution dispenser 22 a given amount of dissolved sample solution 37A located in the rack 21 shown in Fig. 11 and Fig. 18 to inject the dilution solution from the dilution dispenser 22 into the empty sample tube 37B. In the drawings, reference numeral 37C is a sample tube for control use.

In the diluting operation, the primary assay, or the secondary assay is selected, and the number of the samples are input so that the robot 20 is operated as described hereinabove. The 63 sample tubes 37 at maximum are arranged on the rack 21, the number of the samples per cycle is 24 in the primary assay shown in Fig. 18(A), 12 in the second assay shown in Fig. 18(B) for the analysis of the control at the rate of one of four. In the diluting operation, the multiple diluting method is adopted of 10 times in primary assay, 10 times, 100 times, 1,000 times in the secondary assay. The dispenser 22 is set with syringe 100 μℓ, suction amount 100 μℓ on the sample side, Vol. mode, syringe 1 mℓ, suction amount 0.9 mℓ on the reagent side.

(2) The sampling operation is performed after the dilution. Namely, the holder 33b of the nozzle 33a of the sampling pipetter 23 is grasped with the chuck 20b of the robot 20 for sampling the diluted sample at 10 μℓ respectively from the sample tube 37 after the dilution in the rack 21 to inject it into the empty reaction tube 38 arranged in the rack in the incubator 24. During the process, the sampling pipetter 23 is set with syringe 50μℓ, suction amount 10μℓ, on the sample side, Vol mode, syringe 1 mℓ, suction amount 0.1mℓ on the reagent side. In this method, the washing operation of the flexible pipe is completely performed as the reagent is discharged after the discharging of the sample. The external side of the flexible pipe is washed with the tip end of the flexible pipe being dipped after each sampling in the washing vessel 28a disposed on the nozzle stand 28.

The diluted sample in the reaction tube 38 is stirred through the rotor disposed in advance in the reaction tube 38 by the magnetic stirrer 41 after the injection.

(3) Then, addition of the cell suspension is performed. Namely, the holder 34b of the nozzle 34a of the dispenser 25A is grasped with the chuck 20b of the robot 20 to move it to each reaction tube 38 n the incubator 24 to add at 1 mℓ the prepared RBL-1 cell suspension into the reaction tube 38. Thus, the dispenser 25A is set with 0 on the sample side, Vol mode, syringe 2.5 mℓ, syringe capacity 1 mℓ, capacity 0.4 on the reagent side. Immediately before the cell is injected, the timer of the controller is set to 5 minutes to prepare the preincubation so that the cell suspension is injected for the preincubation for five minutes.

(4) Then, the addition of the arachidonic acid is performed. When the timer becomes zero (the preincubation is completed), the timer is set to 15 minutes again for the next reaction to start the injecting of the arachidonic acid. Namely, the holder 35b of the nozzle 35a of the dispenser 25B is grasped with the robot 20 to move it to the reaction tube 38 to inject the arachidonic acid 50 μg + calcium-ionofor A-23187 1 mg/100 μℓ. The dispenser 25B at this time is set, with 0 on the sample side, Vol mode, syringe 250 μℓ, syringe capacity 1 mℓ, capacity 0.4 on the reagent side, to inject 0.1 mℓ (0.25 mℓ x 0.4).

(5) Then, the addition of the ethanol is performed.

When the timer becomes zero (the reaction is completed) after 15 minutes, the ethanol is injected by 1 mℓ to stop the reaction. Namely, the nozzle 36a of the dispenser 25C is grasped with the chuck 20b of the robot 20 to move it to the reaction tube 38 to inject 92 % ethanol, at 1.2 mℓ, containing the GEDTA 1mg. At this time, the dispenser 25C is set with 0 on the sample side, Vol. mode, syringe 2.5 mℓ, syringe capacity 1 mℓ, capacity 0.4 on the reagent.

(6) The reaction mixture preservation is performed after the reaction. Namely, the reaction tube 38 in the incubator 24 is grasped with the chuck 20b of the robot 20 to place it in the preservation vessel 26 to preserve the reaction mixture.

The operation analyzes the lipoxygenase reaction by the use of the RBL-1 cell. In order to analyze the cyclooxigenase reaction, rat platelets, instead of the RBL-1 cell, is used. Blood is drawn from the rat abdominal aorta with the use of 3.2 % sodium citrate to centrifuge it at the room temperature. The rat platelets are prepared so that the concentration of the platelets becomes 1 x $10^9$ cell per mℓ.

The analysis of the cyclooxygenase reaction is similar to that of the lipoxygenase reaction. The prepared platelets is added to the reaction tube 38 from the dispenser 25A. Namely, the platelets of 0.25 mℓ is added to the diluted sample of 10 μℓ in the reaction tube 38. After five minutes preincubation, the arachidonic acid of 125 μg per 25 μℓ is added from the dispenser 25B to perform the reaction for fifteen minutes. Then, the ethanol of 1.0 mℓ is injected from the dispenser 25C to stop the reaction.

Although any enzyme may be used in the present reaction, something showing biological reaction is preferable with the substrate and the product of the enzyme reaction being extremely small in amount. For example, the lipoxygenase or the cyclooxygenase is preferable with the products being leukotrienes, prostaglandins, thromboxanes. The refined enzyme preparation is used, needless to say, as these enzymes. Such tissue cells themselves as described in the embodiment may be used.

The automatic filtration, concentration and injection apparatus shown in Fig. 3 uses a filtrating system for on-line use, instead of the conventional centrifugation system, to remove the admixture such protein or the like. A system for continuously winding the filter paper which is simpler, more inexpensive in apparatus among the filtration system, i.e., a system of having band-shaped filter paper placed on the passage, taking it up after the filtration to replace it with the new paper are adopted. Also, in the concentration, the present system adopts the concentration method with a precolumn, because the reversed phase HPLC is used in the identification, estimation of the products.

Namely, the preliminary column (concentration column) small size for the sample adsorption use is disposed before the analysis column, the sample is dissolved in solvent which is higher in polarity as compared with the eluate. When it is moved through the column the object material is adsorbed. When it is continuously guided into the analystic column from the concentration column with the eluate, the concentration may be performed by the on-line at the same time with the separation/quantitative. The sample of 1 mℓ is injected with the use of the concentration column, so that the sensibility increase of 20 through 50 times is caused in the injection amount (20 through 50 μℓ) to the conventional HPLC. Also, the filtration apparatus is coupled to the concentration apparatus, furthermore an HPLC injecting apparatus is mounted by a six-way valve switch to become the FCI. A series of operation for filtration → concentration → injection to the HPLC may be automatically effected as the apparatus.

Concretely, the apparatus comprises a sampling needle 46 for sampling the reaction mixture from the reaction tube 38, a syringe pump 47 for sucking or discharging the solution drawn from the reaction tube 38 with the sampling needle 46 and the water in the tank 55, a capacity adjusting loop 48 for introduction of the sampling reaction mixture, a filtration unit 49 for filtering of the reaction mixture to be fed from the capacity adjusting loop 48, by a filter paper continuous winding type, a six-way valve 50 with a sampling loop to which the filtered reaction mixture is fed, a water pump 51 for feeding the reaction mixture in the loop of the six-way valve 50A with the water, a six-way valve 52 with a concentration column to which the reaction mixture is fed by the water pump 51, and an analytic pump of HPLC for feeding the eluate to the concentration column 52B to inject into the analytic column the sample solution in the concentration column.

The above-described sampling needle 46 has the vertical operation range of 10 cm to simplify the interlocking operation with the robot 20. The filtration unit 49 has the roll-shaped filter paper that is 50 m in length, 50 mm in width, 0.45 µm in the hole diameter so that it may be continuously used about 3,000 times. also, the capacity adjusting loop 48 and the sampling loop 50B are externally disposed for easier variation in the solution amount.

The respective apparatus is connected through the respective coupling passages 53A through 53M as shown in Fig. 19 of the operation principle view and has electromagnetic valves 54A through 54D disposed on the coupling passages 53A through 53D. In Fig. 20, a water storage vessel 55 is sucked by the syringe pump 47, and the sample mixture is injected into the analytic column from the six-way valve 52 with a concentration column on it by the analytic pump.

The operation of the present apparatus is adapted to be all effected with a control panel 56, shown in Fig. 20, disposed on the upper portion. In Fig. 20, a power switch 57 is provided, and a switch 58 may set the time, which is required to guide the sample into the concentration column 52B from the sampling loop 50B. The time of the switch 58 may be set at the interval of one minute. A switch 59 for setting the analytic time may enter the conditioning of the concentration column automatically after the analytic time to prepare for the next analysis. A switch 60 may set the washing frequency of the passage. A manual, automatic change-over switch is operated in such a manner that, when the operation is switched into the manual one, the manual operations such as vertical operation of the syringe, passage washing, filter paper feeding, water pump starting and stop, six-way valve switch and so on may be performed by the button of (●) in the drawings. In the drawing, (◎) is a start button during the automatic operation, (⦿) is a contact button for transmitting the signals to the external apparatus.

The FCI is operated in the order shown in the block diagram of Fig. 21, which operation will be described hereinafter.

First, the reaction tube 38 with the reaction mixture in it is grasped by the robot 20 and is placed under the sampling needle 46. The sampling needle 46 is lowered and is put into the reaction tube 38. At the same time, the electromagnetic valve 54C disposed on the inter-locking passage 53C between the loop 48 for capaçity adjusting operation and the syringe pump 47 is closed and the electromagnetic valve 54D on the interlocking passage 53D between the water storage vessel 55 and the syringe pump 47 is opened to suck an optional amount of water into the syringe pump 47. Then, the electromagnetic valve 54D is closed, the electromagnetic valve 54A and the electromagnetic valve 54C disposed on the interlocking passage 53A between the loop 48 for capacity adjusting operation and the sampling needle 46 are opened to suck a constant amount the reaction mixture in the reaction tube 38 from the sampling needle 46 to guide it into the loop 48 for capacity adjusting operation. After the suction of a small amount of air, the electromagnetic valve 54A is closed and the electromagnetic valve 54B disposed on the interlocking passage 53B between the loop 48 for capacity adjusting operation and the filtration unit 49 is opened to guide the reaction mixture in the loop 48 for capacity adjustment use into the six-way valve 50 with a sampling loop on it through the filtration unit 49 by the syringe pump 47. In the filtration unit 49, the roll-shaped filter paper of 0.45 μm in hole diameter is continuously wound to filter the sample mixture. The reaction mixture which has been guided into the six-way valve 50 with a sampling loop on it is guided into the sampling loop 50B through a solid-line circuit in the drawing of the six-way valve 50A and the interlocking passage 53F . Continuously the six-way valve 50A is switched into a dot-line circuit to introduce the water of the water pump 51 into the sampling loop 50B through the interlocking 53I, the dot-line circuit of the six-way valve 50A and the interlocking passage 53G to introduce the reaction mixture in the sampling loop 50B into the six-way valve 52 with the concentration column on it through the interlocking passage 53F, the dot-line circuit of the six-way valve 50A and the interlocking passage 53H. In the six-way valve 52 with the concentration column on it, it is introduced into the concentration column 52B through the solid-line circuit of the six-way valve 52A, the interlocking passage 53J and is adsorbed into the concentration column 52B. After the optional time, the six-way valve 52A is switched into the dot-line circuit to feed the eluate into the concentration column 52B through the interlocking passage 53L, the dot-line circuit of the six-way valve 52A, and the interlocking passage 53K to inject the reaction mixture in the concentration column 52B into the analytic column through the interlocking passage 53J, the dot-line circuit of the six-way valve 52A and the interlocking passage 53M to perform the separation, quantitative.

The operation is performed under the conditions of 3.9 in PH, 240 nm in wave length, 1 mℓ per minute in flow speed with acetonitrile : methanol : water : acetic acid = 350 : 150 : 250 : 1 as the eluate.

After the constant time, the water is introduced into the respective six-way valves 50A, 52A from the water pump 51 through the six-way valve 50A with the sampling loop on it being provided as the dot-line circuit, and through the six-way valve 52A with the concentration column on it being provided as the solid-line circuit to perform the washing operation of the flow passage in the valve and of the concentration column 52B. Also, at the same time the syringe pump 47 is operated to wash the sampling needle 46 and the sampling loop 48.

In a series of operations, the water by the syringe pump 47, the reaction mixture by the sampling needle 46 and the suction amount of the air are optionally varied by the dip switch of the electric circuit.

The representative operation cycles of the electromagnetic valves 54A through 54D, the six-way valved 50A, 52A, the sampling needle 46, the syringe pump 47, the filter unit 49, and the water pump 51 are shown in Fig. 22. The operation up to two minutes is one required for the sampling of the sample, five minutes and its subsequent show the washing operation (twice) of the flow passage.

When it is injected into the HPLC by the above described FC1, the analysis operation is performed by the predetermined, constant programmed HPLC.

However, the column to be used in the HPLC uses YMCA-312 (ODS), the eluate A uses 90% acetonitrile, the eluate B uses acetonitrile/methanol/water/acetic acid (1400:800:900:4).

The results of the HPLC are input into the data processer (not shown) through the digitalization of the analog signals by the A/D converter. Here the waveform processing, content calculation, re-processing and recalculation of the improper waveform processing, memory of the chromatogram and calculation results, preparation of the reports with the inhibition, $IC_{50}$ and so on described on them, and the preparation of the data transfer to the upper computer.

[Experiment of the Adsorption Conditions onto the Concentration Column]

(I) Each (1 mg per mℓ solution) of leukotriene $B_4$ ($LTB_4$), hydroxyeicosatetraenoic acids (HETEs) are diluted with water into 200 times, thus resulting in suction amount 1.5 mℓ, sampling loop 1 mℓ. The flow speed of the water pump 51, the switching time of the six-way valve 52A are varied to examine the influences on chromatogram.

When the pump flow speed is 1 mℓ per minute, the constant peak height is shown at 4 minutes or more in switching time but it becomes smaller in proporation to the switching time at 3 minutes or less. The peak height is constant at 2 minutes or more when the pump flow speed is 1.5 mℓ and 2 mℓ per minute. When it is 3 mℓ or more per minute, the concentration column 52B is short, the pressure loss is low. Thus, there are some fears for the bad influences on the adsorption, which are not investigated. The switching time of the six-way valve 52A is 2 minutes at 2 mℓ per minute in the pump flow speed in the adsorption onto the concentration column 52B of the sample mixture.

(II) The influences of the ethanol concentration in the reaction mixture on the adsorption onto the concentration column is investigated. The $LTB_4$ and HETEs (each 1 mg per mℓ) is diluted with ethanol into 10 times. The ethanol 0, 0.1, 0.4, 0.9, 1.2 and 1.9 mℓ is added into this solution 0.1 mℓ and is produced into 2 mℓ with water to provide reaction mixture (ethanol concentration 5, 10, 25, 67, 100 %), so that the peak height of the HPLC, and the shape are investigated. Fig. 23(a) shows the chromatogram of 5 % in ethanol concentration, Fig. 23(b) one of 50 %, Fig. 23(c) one of 67 %. The chromatogram of 5 %, 50 % is almost the same in both the peak height and the shape with the influences of the ethanol being not recognized, but in 67 %, the peak overlaps and becomes broad so that the normal peak can not be provided. Fig. 24 shows the chromatogram provided in a case where the $LTB_4$ and the HETEs (each 1 mg per mℓ) is diluted with water into 5 times and is directly injected at 50 μℓ into HPLC (UV240 nm). As compared with Fig. 23(b), it is found out that the peak separation degree remains without any change, and the on-line of the FCI has no influences upon the chromatogram in 50 % or less in ethanol. This is similar to a case where acetonitrile is used.

[Experiment of Filter-Paper Selection]

The influences of the filter area and the hole diameter on the filtration are investigated.

The 50 % ethanol suspension (cells 5 x $10^7$ per mℓ) of the RBL-1 is moved through the membrane filter of cellulose nitrate, cellulose acetate mixture type different in diameter and hole diameter at 1 mℓ per minute in filtrating speed, so that the turbidity and the filter paper clogging are investigated after the passing. The filter paper of 13 mm in diameter causes the sudden pressure rise because of clogging at 0.6 through 0.7 mℓ. The smooth filter operation is performed at 25 mm and 47 mm. The filter paper of 25 mm in diameter causes the clogging at 1.5 mℓ. Also, the clogging is caused at 0.3 through 0.5 mℓ with the hole diameter of the filter paper being 0.22 μm, but the easy filtering operation is performed at 0.45 μm or more. However, at 1.2 μm, the turbidity is caused in the passing solution.

Then, influences of the material for the filter paper on the chromatogram is investigated. The HPLC is performed after the passage of the filtration/concentration apparatus with 50 % ethanol suspension (cells 5 x $10^7$ per

mℓ) of the RBL-1 being the sample solution. The filter paper of 0.45 through 0.6 μm in hole diameter, 25 mm in diameter is used. Also, the sample solution is centrifuged (2500 rpm, ten minutes) and the supernatant is used for control. The filter paper is of 0.45 μm in the hole diameter of the RC of a reproduction cellulose type, 25 mm in diameter, filter area. Although the leakage from the filter paper can hardly be recognized in the case of water, a large amount of leakage can be recognized in the case of the 50 % ethanol.

[Precision and Comparison Experiment with the Conventional Method]

The $LTB_4$ and HETEs (each 1mg per mℓ) is diluted into 10 times with ethanol. The ethanol 0.9 mℓ is added into this solution to provide 2mℓ, with water, as the sample solution. It is injected into FCI to perform the HPLC. The accuracy is repeatedly obtained from the peak height provided. The accuracy as good as 0.4 through 1.7% in coefficient of variation as shown in the following Table 1.

Table 1

| | Peak Height (mm) | | | | | |
|---|---|---|---|---|---|---|
| Times | $LTB_4$ | 15-HETE | 11-HETE | 8- and 12-HETE | 9-HETE | 5-HETE |
| 1 | 52.8 | 94.8 | 93.2 | 80.3 | 40.8 | 50.6 |
| 2 | 54.0 | 95.7 | 97.5 | 80.8 | 41.5 | 51.9 |
| 3 | 53.1 | 95.3 | 95.0 | 80.4 | 41.2 | 50.9 |
| 4 | 54.7 | 95.5 | 96.3 | 80.5 | 41.0 | 51.0 |
| 5 | 54.4 | 94.7 | 94.2 | 79.8 | 40.9 | 50.8 |
| 6 | 54.2 | 94.6 | 94.3 | 80.4 | 41.0 | 50.9 |
| Mean | 53.9 | 95.1 | 95.1 | 80.4 | 41.1 | 51.0 |
| Standard Deviation | 0.75 | 0.46 | 1.57 | 0.33 | 0.25 | 0.45 |
| Coefficient of Variation | 1.39 | 0.48 | 1.65 | 0.41 | 0.69 | 0.89 |

In the Table 1, UV 240 nm is employed to measure except for $LTB_4$. Also, the present method is conformed to the conventional method, in accordance with the investigation, by the use of RBL-1, of the interference of 5-lipoxygenase of unique raising interference agent 2-(12-hydroxy-dodeca-5, 10-diynyl)-3, 5, 6-trimethyl-1, 4--benzoquinone (AA-861).

The injection amount of the reversed phase HPLC using the column of 4 through 4.6 mm in inner diameter is 50 through 100μℓ at limit. Though the influences against the separation is feared in the method of injecting the sample mixture 1 mℓ into the pre-column to introduce it into the analytic column, no influences are recognized even at 50% ethanol as shown in Fig. 24(b). The concentration method by the pre-column is the same as the case where 1 mℓ is injected directly into the HPLC, so that the concentration of 10 through 20 times may be performed under the on-line. Also, the repeating accuracy is better with the results at the AA-861 is conformed to the conventional method.

[Chromatograph Operated in Accordance With Operation System With the Use of the Present Invention Apparatus, and Comparison Experiment Results in the Conventional Manual Method]

The chromatogram obtained through the operation by the use of the RBI-1 cell, and the chromatogram of the case where the AA-861 is $10^{-6}$M added are shown in Fig. 25. The chromatogram by the manual method of the $LTB_4$ and the HETE isomer is shown in Fig. 26. The references 1, 2, 3, 4 of Fig. 25 come from Fig. 26. Although the retention time of the automatic method and the manual method remain unchanged, the number of the theoretical plates showing the column performance is 11500 (stage/column) with automation in 5-HETE, 8100 in the manual method. The number of the theoretical plates in the automatic method is larger, because of the time control for the valve switching operation improved through the automation and the concentration column, the analytical column. The repeating accuracy is improved by about one half, $LTB_4$ 3.5%, 5-HETE 4.0%, in the coefficient of variation as compared with the manual method. The reason why the coefficient of variation is improved is that the time for the reaction, the injection and so on becomes correct through the automation and the injection liquid amount becomes correct. Also, the time for requiring the pretreatment is shortened into one hour and twelve minutes through one hour and sixteen minutes, about 1/3 of the manual method in the number of the largest samples (primary assay 24, the secondary assay 12, number of the processings 61), thus saving a large amount of time. Although the operation analyzes the lipoxygenase reaction by the use of RBL-1 cell, a rat platelets, instead of the RBL-1 cell, is used in the analysis of the cyclooxygenase reaction. The rat platelets are prepared so that the platelets concentration may become 1 x $10^9$ cell per mℓ through the drawing of the blood from the rat abdominal aorta with the use of 3.2 % sodium citrate to centrifuge it at the room temperature.

The analysis of the cyclooxygenase reaction is similar to the analysis of the lipoxygenase reaction. The prepared platelets is added into the reaction tube 16 from the dispenser 6. Namely, the platelets of 0.25 mℓ is added to the diluted sample of 10μℓ in the reaction tube 16. After the reaction mixture is preincubated for 5 minutes, arachidonic acid of 125μg per 25 μℓ is added from the dispenser 7 to incubate for 15 minutes to stop the reaction through the injection of the ethanol 1.0 mℓ from the dispenser 8.

Though any enzyme to be used in the present reaction will do, particularly the substrate of the enzyme reaction or the product in small amount which shows the biological activity is preferable. For example, the lipoxygenase or the cyclooxygenase where the products are leukotrienes, prostaglandins, thromboxanes is preferable. As the enzyme preparation, such tissue cells as described in the embodiment may be used in addition to purified enzymes.

As is clear from the above-described description, according to the automatic apparatus of the present invention, the saving is provided, the time may be considerably shortened to 1/3 of that of the conventional manual operation because of the automation of the operation from the pretreatment steps to the data processing and the analysis. Also, the coefficient of variation are reduced into 1/2 in the accuracy of the measurement value as compared with the conventional manual method because of the corrector injection amount or the like.

Also, each member of this apparatus has the following effects.

1. As the robot may be used like the human hand, all the complicated operations may be performed, and the varied operation may be simply performed. Particularly, the use of the horizontal multi-joint type robot allows faster speed, better precision, wider action range and simplifies easier assembling of the multiple-object operation method.

2. In the automatic weighing, dissolution apparatus, the sample tube is grasped and moved with the chuck of the robot. In the automatic dilution reacting apparatus, the nozzle holder is grasped and carried with the chuck of the robot, but the sample tube and the reaction tube are not moved, so that the respective time may be shortened, and particularly the speed of the time for the enzyme reaction may be improved.

3. As the various types of dispensers, an automatic dispenser composed of digital diluter/pipetter is used, so that the addition, dilution, sampling, injection of the solvent may be performed in shorter time and with better precision.

4. As the washing operation within the nozzle is effected simultaneously with the injection of the diluted solution in the automation dilution reacting apparatus, the washing operation of the nozzle is not required to be effected for each of the samples. When the washing is required, the washing may be performed with better efficiency, because the washing vessel may be formed integrally with the nozzle stand.

5. As the stirring is performed by one magnetic stirrer with a rotor being kept in advance in the reaction tube in the automatic reaction apparatus, the stirring of a plurality of reaction tubes 64 may be performed at one time.

6. As the filtration, the concentration, and injection to the HPLC of the reaction mixture are performed with the full automation under the on-line, the operation time may be considerably shortened. Also, the quantitative property is improved through the automation, thus resulting in better accuracy of 0.4 through 1.7 % in coefficient of variation. In accordance with this invention, the sample mixture is filtered by the filtration system, instead of the centrifugation system. As the filter paper continuous winding system is adopted, it is suitable for the on-line so that the equipment expenses may be reduced. In addition, as the milli-pore filter of 0.45 μm is used for the filter paper, the filtration at the HPLC becomes unnecessary. The operation for evaporation of the sample mixture may be made unnecessary through the passing operation through the six-way valve with the concentration column on it prior to the analytical column. Besides, according to the concentration column, the concentration 10 times through 20 times as many as the conventional one may be performed, the repeating accuracy may be good, thus resulting in various advantages.

Although the present invention has been fully described by way of example with reference to the accompanying drawings, it is to be noted here that various changes and modifications will be apparent to those skilled in the art. Therefore, unless otherwise such changes and modifications depart from the scope of the present invention, they should be construed as being included therein.

What is claimed is:

1. An automatic analysis method of enzyme reaction for automatically carrying out all the steps of weighing operation of a sample, adding operation of solvent to the sample, dissolving operation of the sample in the solvent, adding operation of the solvent into the sample solution, injecting operation of the diluted sample solution into a reaction tube, adding operation of enzymes into the reaction tube to produce a reaction mixture, the moving operation of the reaction tube onto preservation vessel, filtering operation of the reaction mixture into the reaction tube to produce a filtrate, concentrating operation of the filtrate, injecting operation of the filtrate into the analysis apparatus, and processing operation of the analysis data with respect to the filtrate.

2. An automatic analysis method of enzyme reaction, in accordance with Claim 1, wherein the weighing of a sample, adding of a solvent, dissolution are automatically carried out by all the steps of grasping a sample tube retained in a rack with a chuck provided on a movable arm of an automatic robot for moving the sample tube to an electronic balance, a solvent dispenser, a dissolution vessel, adding the solvent predetermined in accordance with the weighing value of sample after weighing operation of the substance by the electronic balance, placing the sample tube into a dissolution vessel after the addition of a solvent to dissolve the sample, moving the sample tube with the dissolved sample in the rack for dilution provided in a reaction apparatus of the next step, the addition of solvent, injection into the reaction tube, addition of the enzyme reaction mixture are carried out by the steps of grasping each nozzle of a diluted solution dispenser, a sampling pipetter and an enzyme reaction mixture dispenser with the chuck of the automatic robot and moving the nozzle and injecting into the sample tube retained in the rack for dilution use and the reaction tube retained in the rack placed in the incubator for performing injecting operation, adding dilution solvent from a diluted solution dispenser into the sample tube in a dilution rack to perform the diluting operation into given times, injecting diluted solution of the sample tube from the dispenser to inject it into the reaction tube, sequentially injecting the enzymes and factors necessary for enzyme reaction into the reaction tube at the intervals of given time to start the reaction, grasping the reaction tube by the robot to move onto the preservation vessel after the reaction, grasping the reaction tube retained in the preservation vessel by the robot to place the reaction tube in the position of a reaction mixture sampling needle disposed in a filtration/concentration/injection (FCI) apparatus, filtering reaction mixture through a filtration unit after sampling operation of reaction mixture with a sampling needle, feeding the eluate into a column for concentration use after the adsorption of a filtrate onto a column for the concentration use to inject into an analytical column of a high performance liquid chromatograph so that the obtained data is processed with a processing apparatus, and the operation of the robot is automatically controlled in accordance with a program input into the computer to automatically perform all the steps from weighing to analysis.

3. An automatic analysis method of enzyme reaction in accordance with Claim 1, wherein the enzyme is lipoxygenase.

4. An automatic analysis method of enzyme reaction in accordance with Claim 1, wherein the enzyme is cyclooxygenase.

5. An automatic analysis method of enzyme reaction in accordance with Claim 1, wherein solution for enzyme reaction of the cell including the lipoxygenase or the cyclooxygenase, the arachidonic acid, and the ethanol is provided by adding sequentially, at the intervals of the given time.

6. An automatic weighing and dissolving method for samples, comprising the steps of retaining in a rack a plurality of sample tubes for free taking-out operations in different positions on the periphery of an automatic robot, an electronic balance, a solvent adding apparatus, a dissolution vessel, so that the sample tube in the rack into which a sample is thrown is freely grasped with a chuck disposed on a movable arm of the automatic robot to sequentially move it automatically onto the electronic balance, the solvent adding apparatus and the dissolution vessel,

and employing a computer for controlling the operation of said robot, setting a solvent adding amount calculated in accordance with the weight of the sample weighed by an electronic balance, adding the solvent into the sample tube, for automatically controlling each of the apparatuses such as dissolution vessels into which the sample tube after the addition of solvent is placed.

7. An automatic dilution reacting method comprising the steps of retaining in a rack with a plurality of sample tubes, a dilution dispenser, a sampling pipetter, a incubator having a plurality of reaction tubes disposed in the incubator, a nozzle washing vessel disposed on positions different on the periphery of an automatic robot, nozzles mounted respectively through the flexible tubes on each of the dispensers, these nozzles being detachably placed on the nozzle stand provided within the operation range of the robot while each of the nozzles is freely grasped with a chuck provided on a movable arm of the robot so that the nozzle may move into the nozzle washing vessel, the opening of the sample tube in a rack or into the opening of the reaction tube in an incubator,

and automatically controlling, by a controller, the robot in a manner that the diluted sample solution is pipetted into the sample tube in a rack from a dilution dispenser and a diluted sample is pipetted from the sample tube by the sampling pipetter to be pipetted into the reaction tube in the incubator, and the reaction mixture is pipetted from a reaction mixture sampling pipetter into a dilution sample in the incubator.

8. An automatic filtration/concentration/injecting (FCI) method comprising the steps of feeding by a syringe pump, sampling a mixture into a sampling solution pipetter from a sample tube with a sampling needle, filtering the pipetted sample mixture by a filtering unit of a filter-paper continuous winding type, controlling a valve with a sampling loop thereon to feed the filtered sample mixture and a valve with a concentration column thereon, feeding by a water pump the sample solution in the loop of the sampling loop valve, together with water, into a concentration column of the valve with a concentration column on it, and feeding through a valve feeding the eluate into the concentration column to inject into an analytical column the sample solution located within the concentration column.

9. An automatic analysis apparatus of enzyme reaction, comprising a means for weighing a sample, a means for adding solvent to the sample, a means for dissolving the sample in the solvent, a means for adding solvent to the dissolved sample solution, a means for injecting diluted sample solution into a reaction tube, a means for adding enzymes into the reaction tube, a means for moving the reaction tube onto a preservation vessel, a means for filtering the reaction mixture in the reaction tube, a means for concentrating the filtrate, a means for injecting into an analysis apparatus, and a means processing the analysis data.

10. An automatic analysis method of enzyme reaction, in accordance with Claim 9, wherein the means for performing weighing operation, adding solvent and performing dissolution comprises a rack for retaining many sample tubes on the different periphery of a robot to be automatically controlled, an electronic balance, a solvent dispenser, a dissolution vessel with an ultrasonicator apparatus thereon so that the sample tube in the rack is grasped with chuck disposed on the movable arm of a robot to move it onto the electronic balance, the solvent adding means, the dissolution vessel,

the dilution reaction means for a resolved sample comprises a rack for dilution use for retaining many sample tubes in the different position on the periphery of the robot to be automatically controlled, a incubator provided with the rack for retaining many reaction tubes, a dilution dispenser, a sampling pipetter, a plurality of dispensers for injecting enzymes, a preservation vessel provided with a rack for retaining the reaction tube, so that a nozzle of each injector is grasped with a chuck of the robot to move it onto the sample tube and the reaction tube to perform the injection, the reaction tube in the reaction vessel is grasped with the chuck of the robot to move it onto the rack of the preservation vessel,

the filtration/concentration/injection (FCI) apparatus comprises a sampling needle for sampling the reaction mixture from the reaction tube through the operation by a syringe pump, a filtration unit of a filter paper continuous winding type for filtering the sampled reaction mixture, a concentration column for concentrating the filtrate, a valve for feeding the eluate into the concentration column to inject it into an analytical column, each of these apparatuses being so disposed that the filtration, concentration, injection is performed on one line.

11. An automatic weighing and dissolution apparatus for samples comprising a rack for retaining a plurality of sample tubes for free taking-out operations in the different positions on the periphery of an automatic robot, an electronic balance, a solvent adding apparatus, a dissolution vessel, so that the sample tube in the rack into which the sample is thrown is freely grasped with a chuck disposed on a movable arm of the automatic robot to sequentially move it automatically onto the electronic balance, the solvent adding apparatus and the dissolution vessel,

and a computer for controlling the operation of said robot, setting the solvent adding amount calculated in accordance with the weight of the sample weighed by the electronic balance, adding the solvent into the sample tube, for automatically controlling each of the apparatuses such as dissolution vessels into which the sample tube after the addition of solvent is placed.

12. An automatic weighing and dissolving apparatus for samples in accordance with Claim 11, wherein a horizontal multi-joint type robot is used as the automatic robot.

13. An automatic weighing and dissolving apparatus for samples in accordance with Claim 11, wherein a plural kinds of solvent is set for the solvent adding apparatus.

14. An automatic weighing and dissolving apparatus for samples in accordance with Claim 11, wherein a heater is mounted in the dissolution vessel with an ultrasonicator apparatus on it so that the heater is adapted to be controlled by the computer.

15. An automatic weighing and dissolving apparatus for samples in accordance with Claim 11, wherein the taking-in or taking-out of the sample tube with respect to the electronic balance is adapted to be controlled by the computer.

16. An automatic weighing and dissolving apparatus for samples in accordance with Claim 11, wherein the amount of solvent and diluted solution into the sample is added in response to the weight of the sample weighed by the electronic balance.

17. An automatic dilution reacting apparatus comprising a rack for retaining a plurality of sample tubes, a dilution dispenser, a sampling pipetter, an incubator having a plurality of reaction tubes disposed in the vessel, a nozzle washing vessel disposed on the positions different on the periphery of an automatic robot, nozzles mounted respectively through flexible pipes on each of the dispensers, these nozzles being detachably placed on a nozzle stand provided in the operation range of the robot while each of the nozzles is freely grasped with a chuck provided on a movable arm of the robot so that it may move into the opening of the sample tube in the nozzle washing vessel and a rack or into the opening of the reaction tube in the incubator,

and a controller for automatically controlling the robot so provided that the diluted sample is pipetted from the sample tube by the sampling pipetter to be injected into the reaction tube in the incubator, and the reaction mixture is injected from the reaction mixture dispenser into the diluted sample in the incubator.

18. An automatic dilution reacting apparatus in accordance with Claim 17, wherein a preservation vessel is provided on the periphery of the robot, and the reaction tube is adapted to be grasped with the chuck of the robot after the reaction by the incubator to be moved into the preservation vessel.

19. An automatic weighing and dissolving apparatus for samples in accordance with Claim 17, wherein a horizontal multi-joint type robot is used as the automatic robot.

20. An automatic dilution reacting apparatus in accordance with Claim 17, wherein a digital diluter pipetter is used as each of the dispensers, and a coil-shaped teflon tube is used as a coupling flexible pipe so that the nozzle is integrally formed at the tip end of the tube.

21. An automatic dilution reacting apparatus in accordance with Claim 17, wherein a dissolution vessel with a magnetic stirrer on it is used as the incubator.

22. An automatic dilution reacting apparatus in accordance with Claim 17, wherein a plurality of dispensers for the reaction mixture different in kind are provided as the reaction mixture sampling pipetter, and the reaction mixture different in kind are sequentially injected into one reaction tube at the intervals of the given time to cause plural kinds of reactions.

23. An automatic dilution reacting apparatus in accordance with Claim 22, wherein at least enzyme solution is used as the reaction mixture.

24. An automatic filtration/concentration/injection (FCI) apparatus comprising a syringe pump for feeding sampling solution pipetted from a sample tube with a sampling needle, a filter unit for filtering by a filter paper continuous winding type the pipetted sample solution, a valve with a sampling loop on it to which the filtered sample solution is fed, a valve with a concentration column on it, a water pump for feeding the sample solution in the loop of the valve with a sampling loop, together with water, into a concentration column of a valve with the concentration column thereon, and a valve for feeding eluate into the concentration column to inject into an analytical column for sample solution in the concentration column.

*Fig. 1*

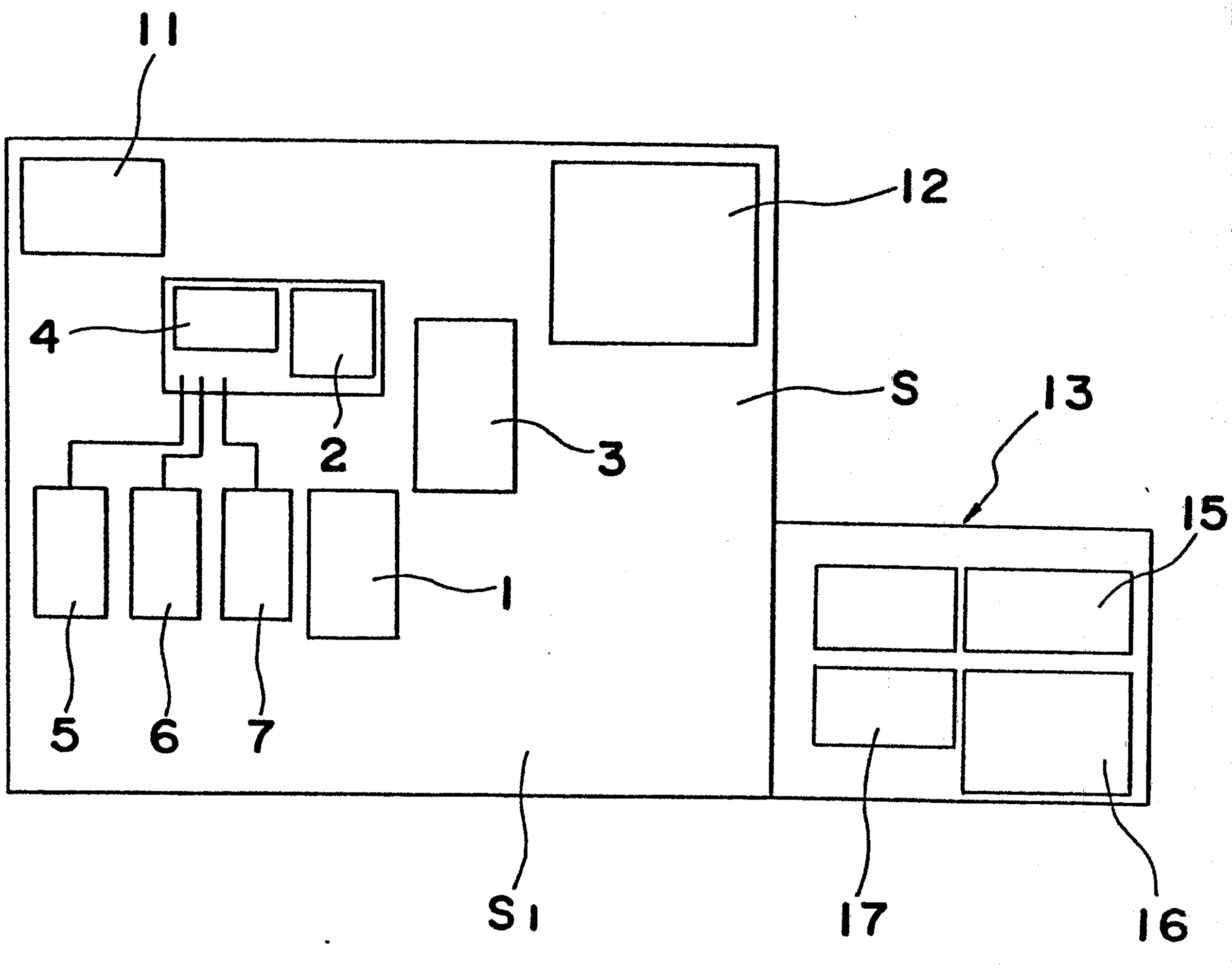

2/26

Fig. 2

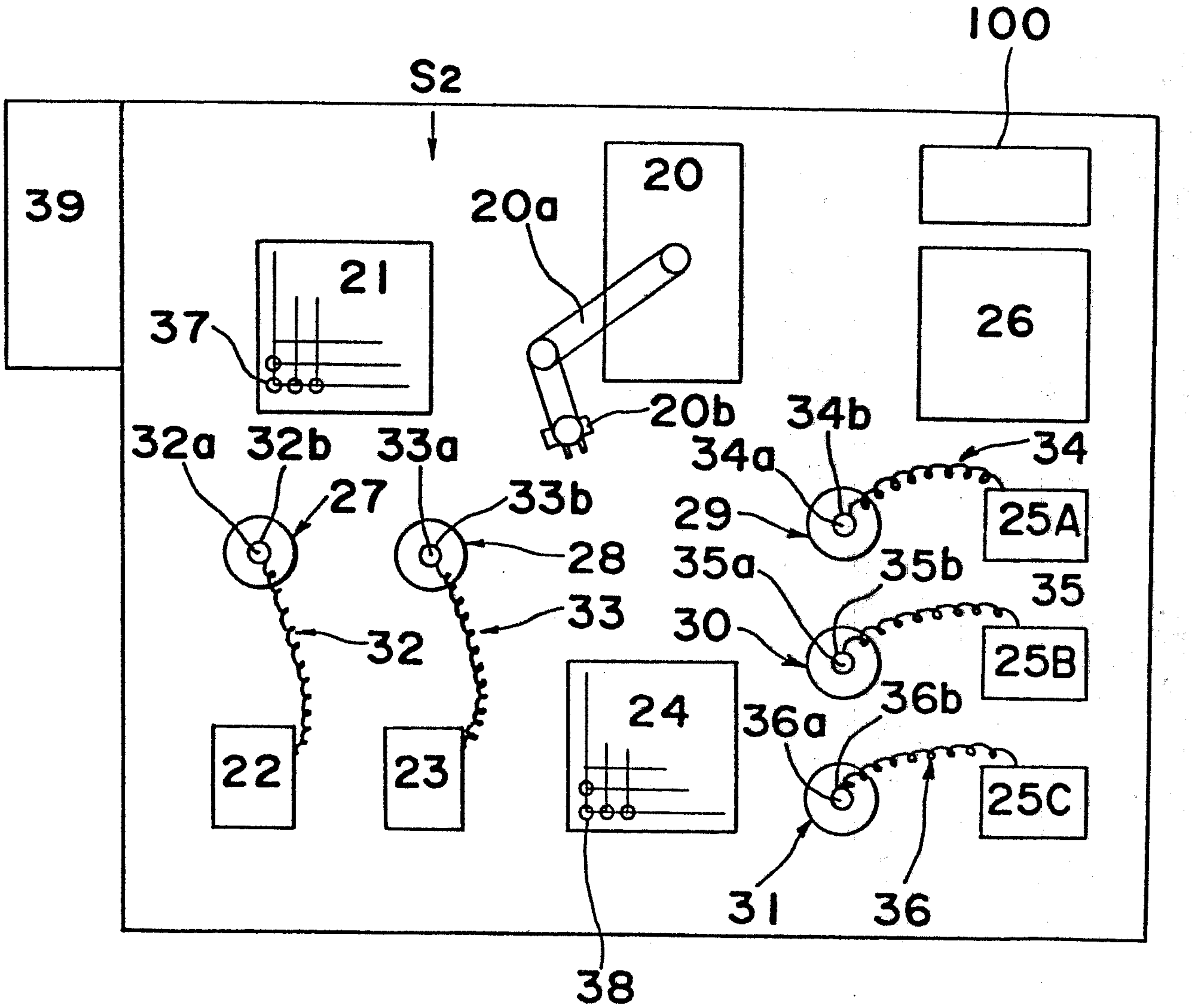
100
S2
39
20
20a
21
26
37
20b
34b
32a
32b
33a
34a
34
27
33b
29
25A
28
35a
35b
35
32
33
30
25B
24
36a
36b
22
23
25C
31
36
38

*Fig. 3(A)*

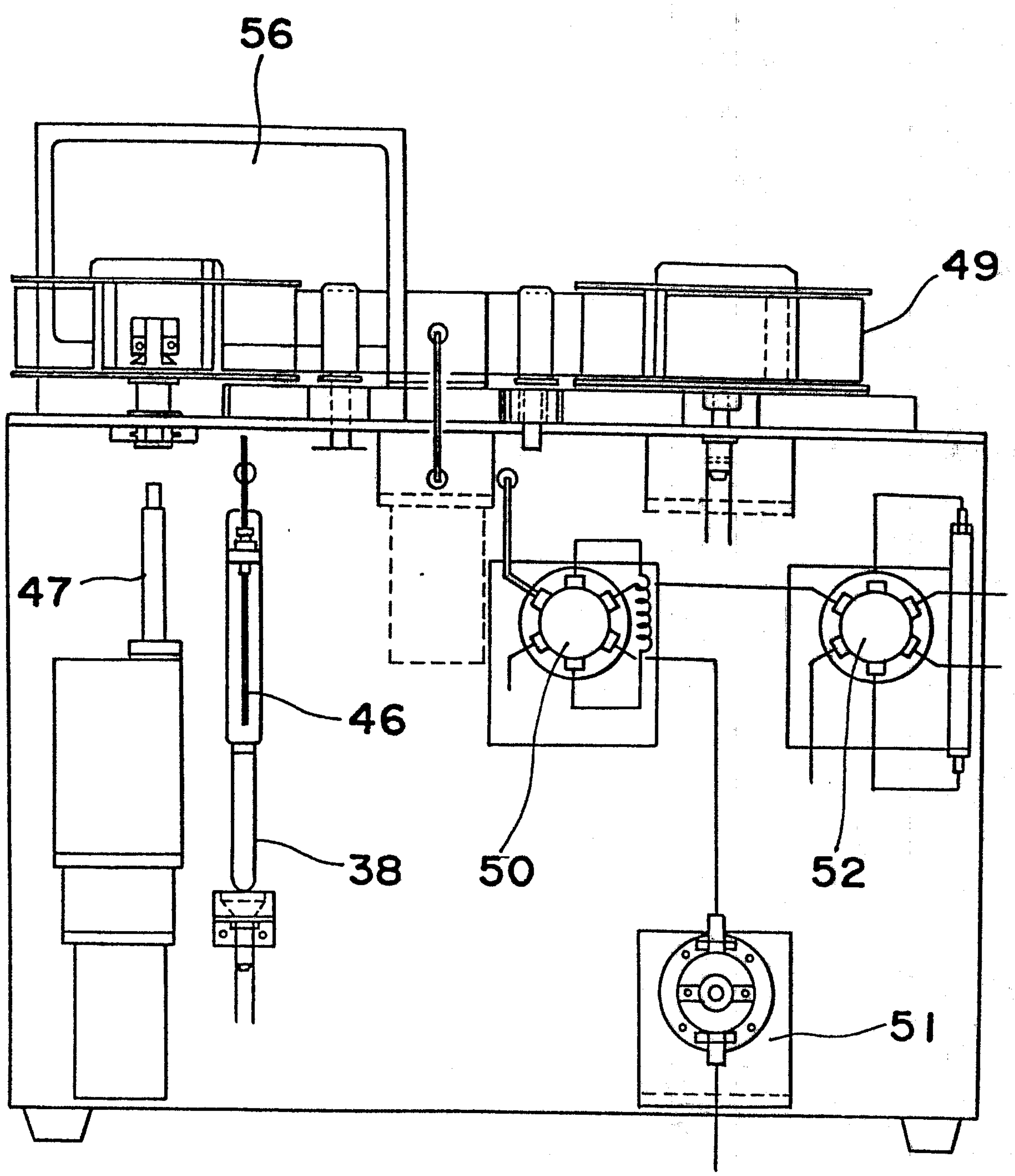

*Fig. 3(B)*

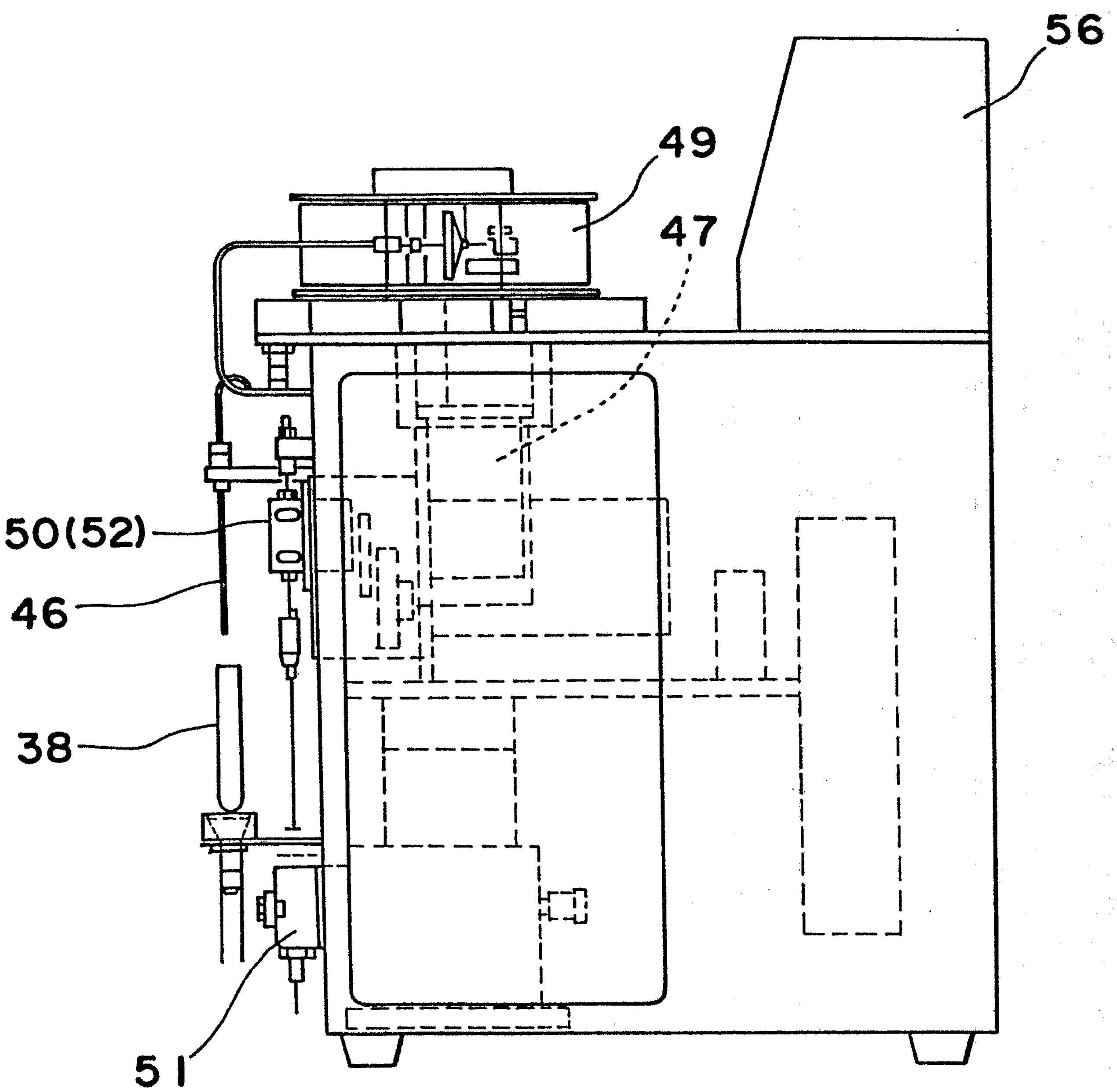

Fig. 4(A)

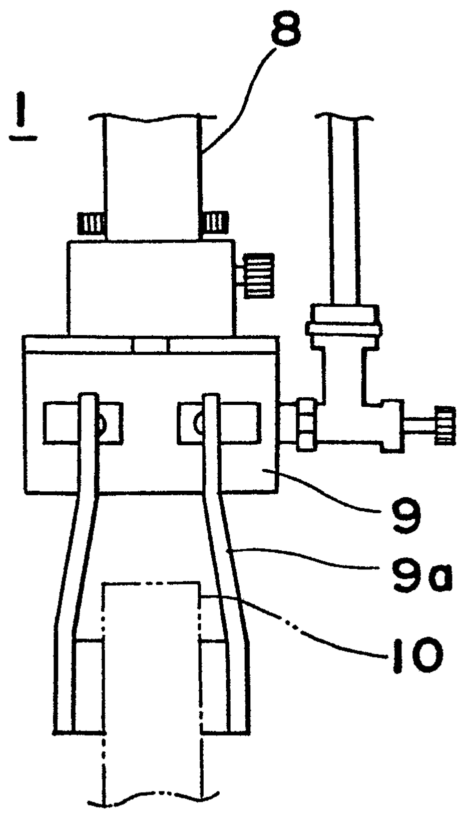


Fig. 4(B)

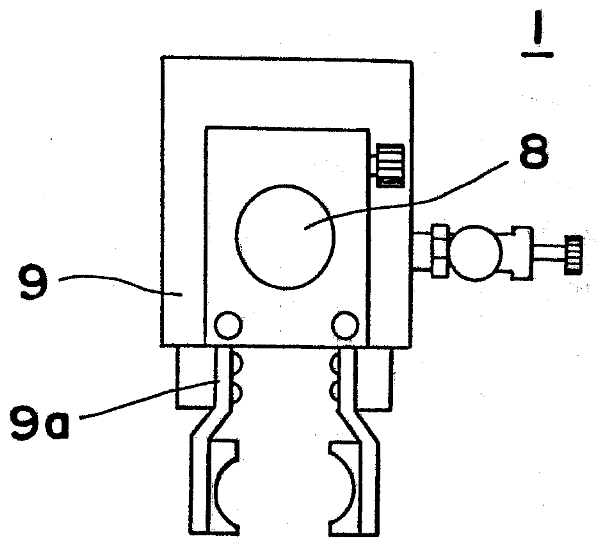


Fig. 6(A)

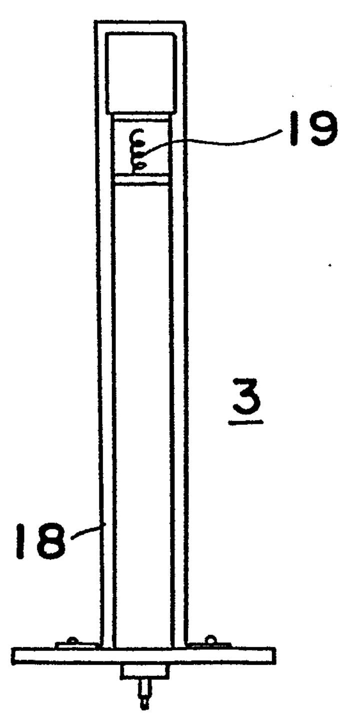


Fig. 6(B)

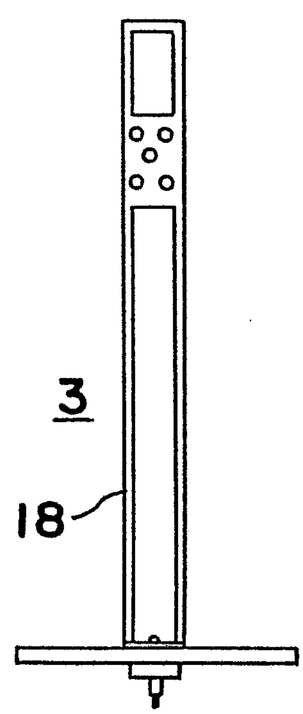


Fig. 6(C)

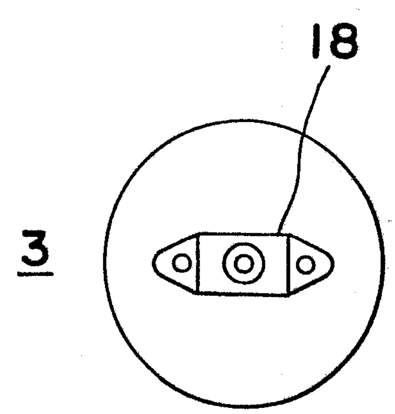

Fig. 5(A)

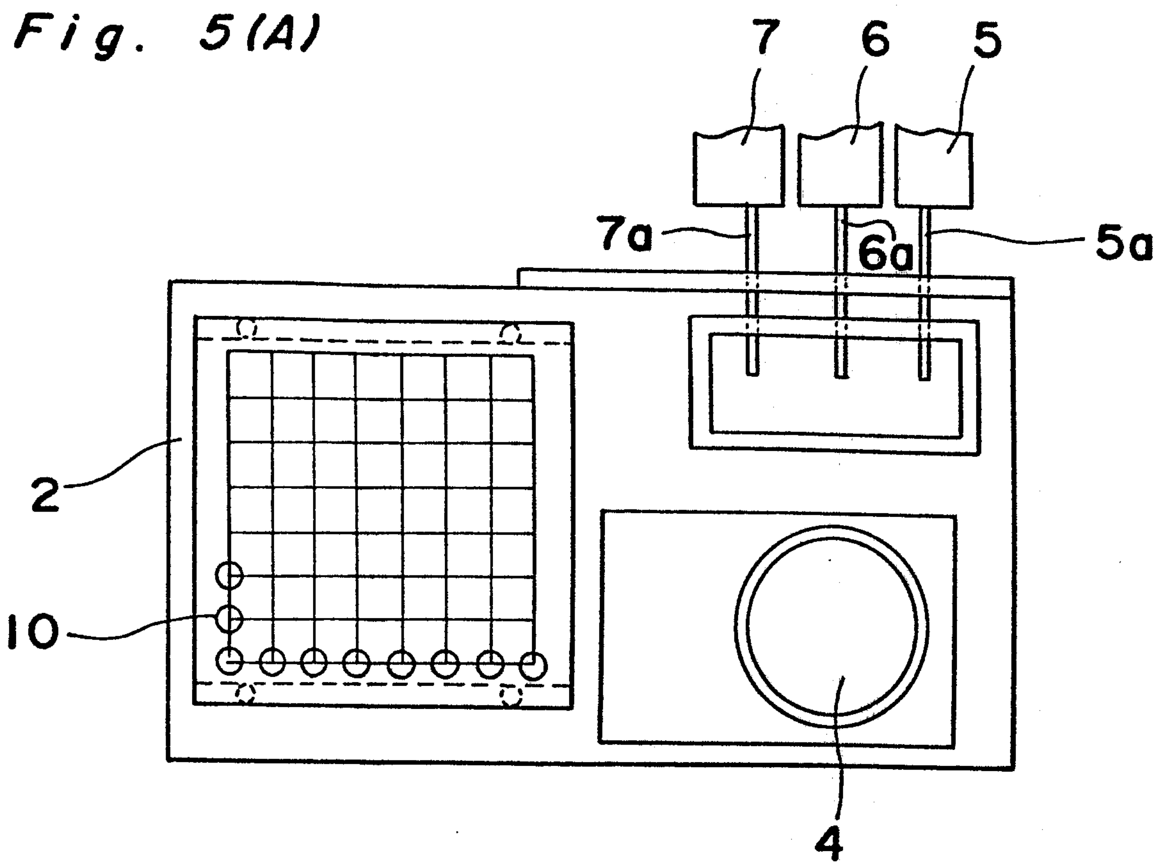


Fig. 5(B)

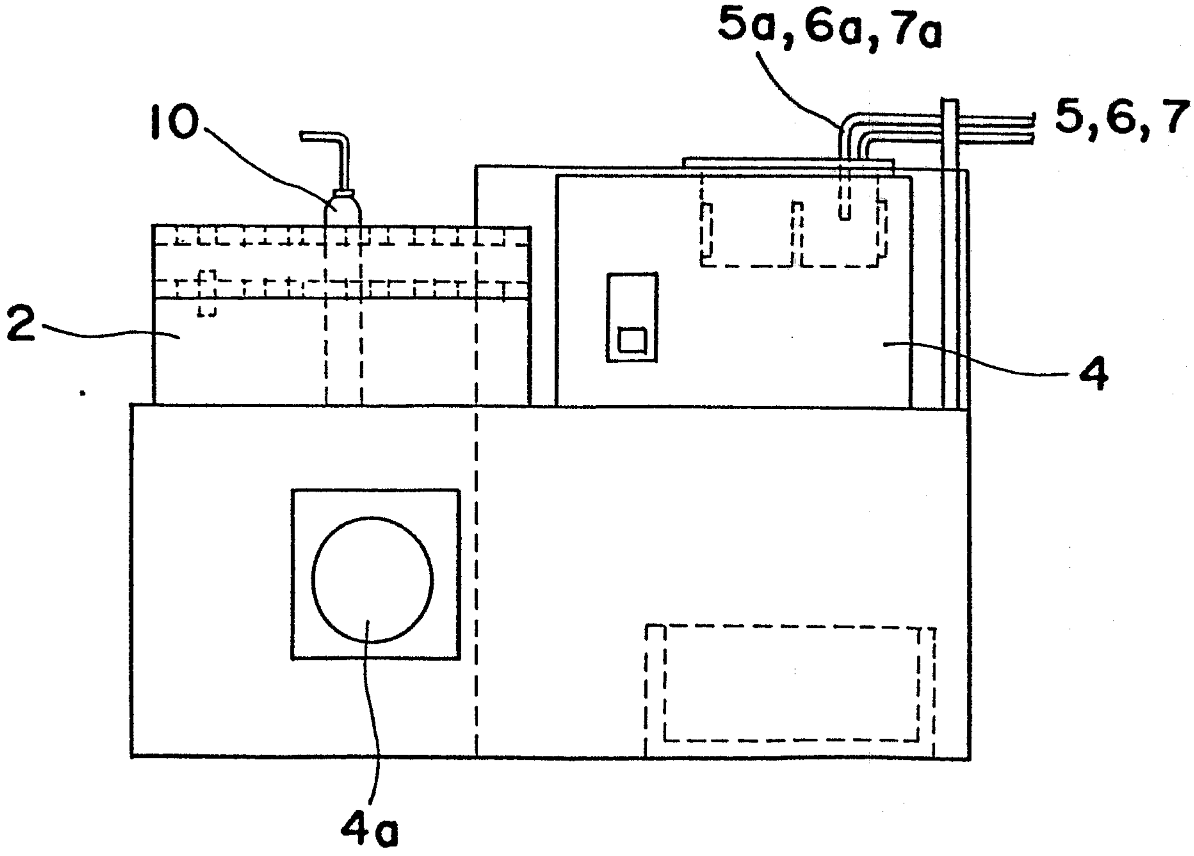

Fig. 7

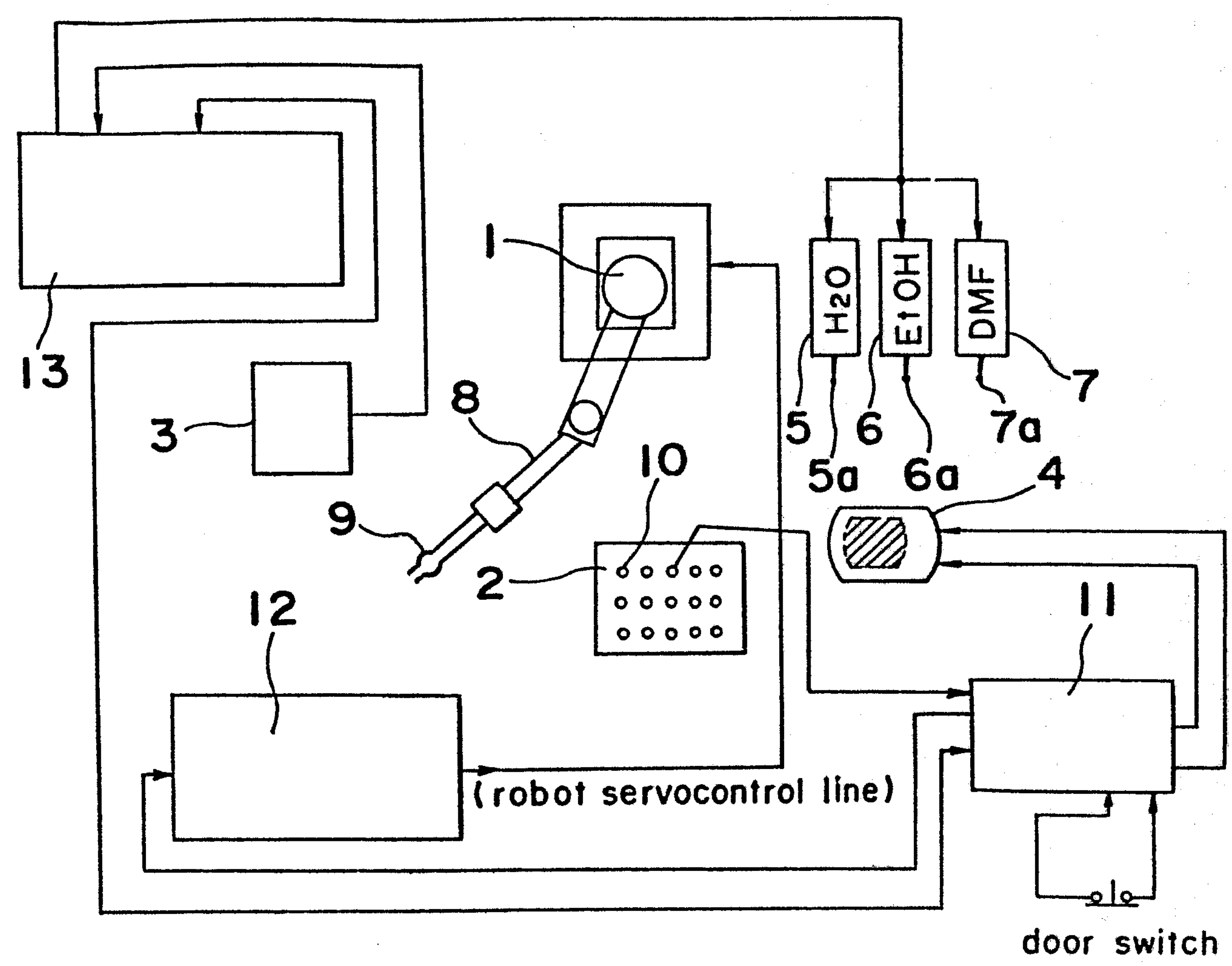
1
13
3
8
9
10
2
12
H2O
EtOH
DMF
5
5a
6
6a
7
7a
4
11
(robot servocontrol line)
door switch

Fig. 8

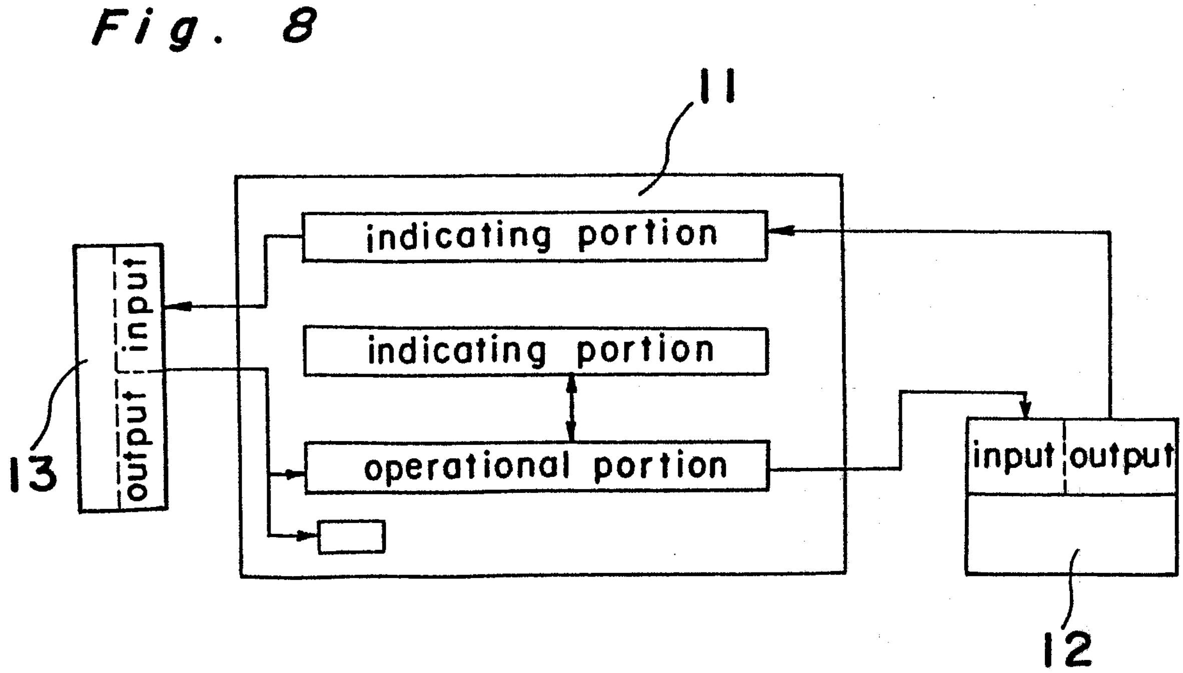
11
indicating portion
indicating portion
operational portion
input
output
13
input
output
12

Fig. 9

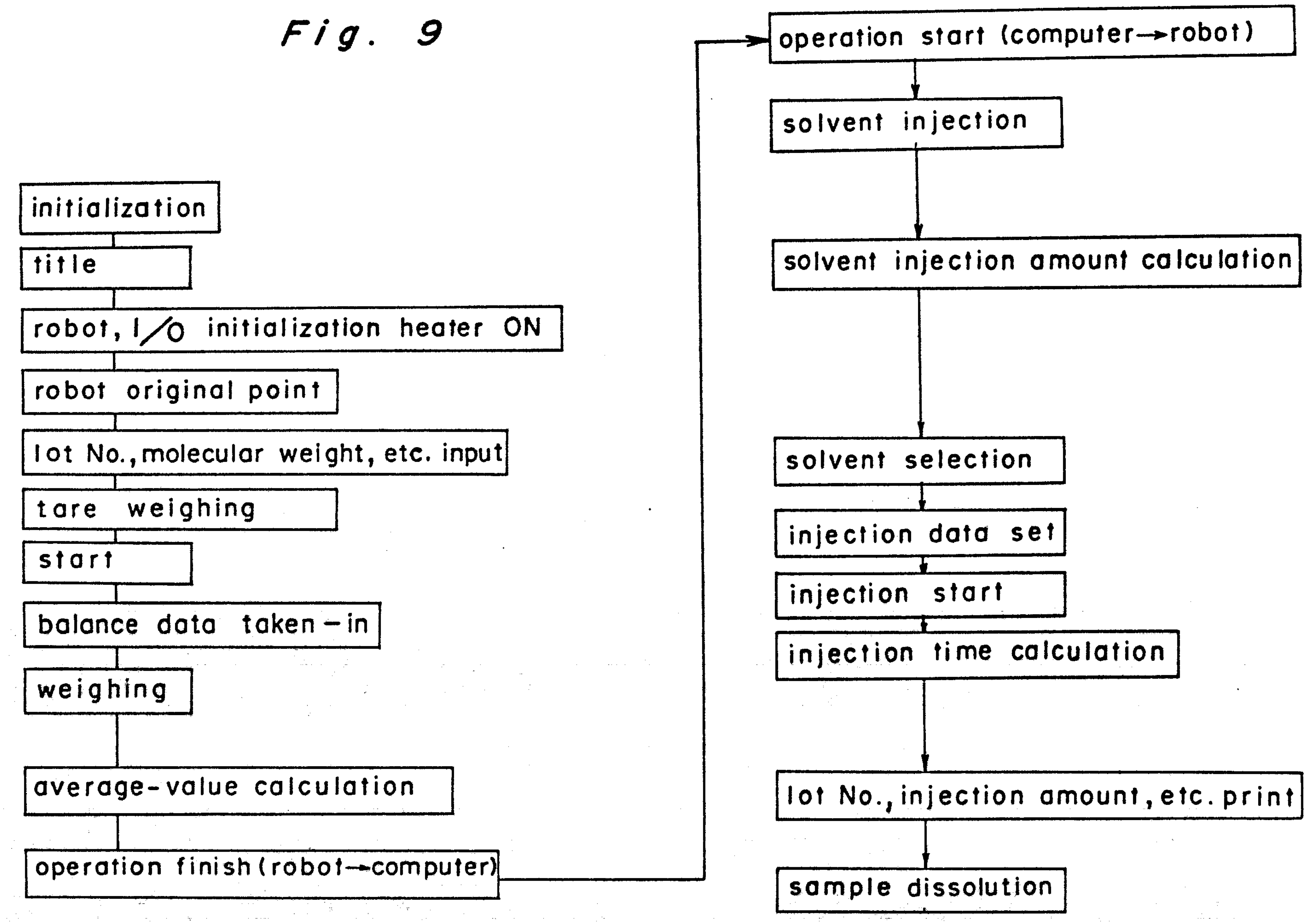
initialization
title
robot, I/O initialization heater ON
robot original point
lot No., molecular weight, etc. input
tare weighing
start
balance data taken-in
weighing
average-value calculation
operation finish (robot→computer)
operation start (computer→robot)
solvent injection
solvent injection amount calculation
solvent selection
injection data set
injection start
injection time calculation
lot No., injection amount, etc. print
sample dissolution

Fig. 10

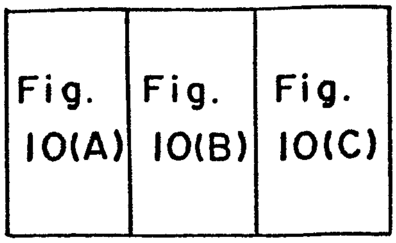


Fig. 10(A)

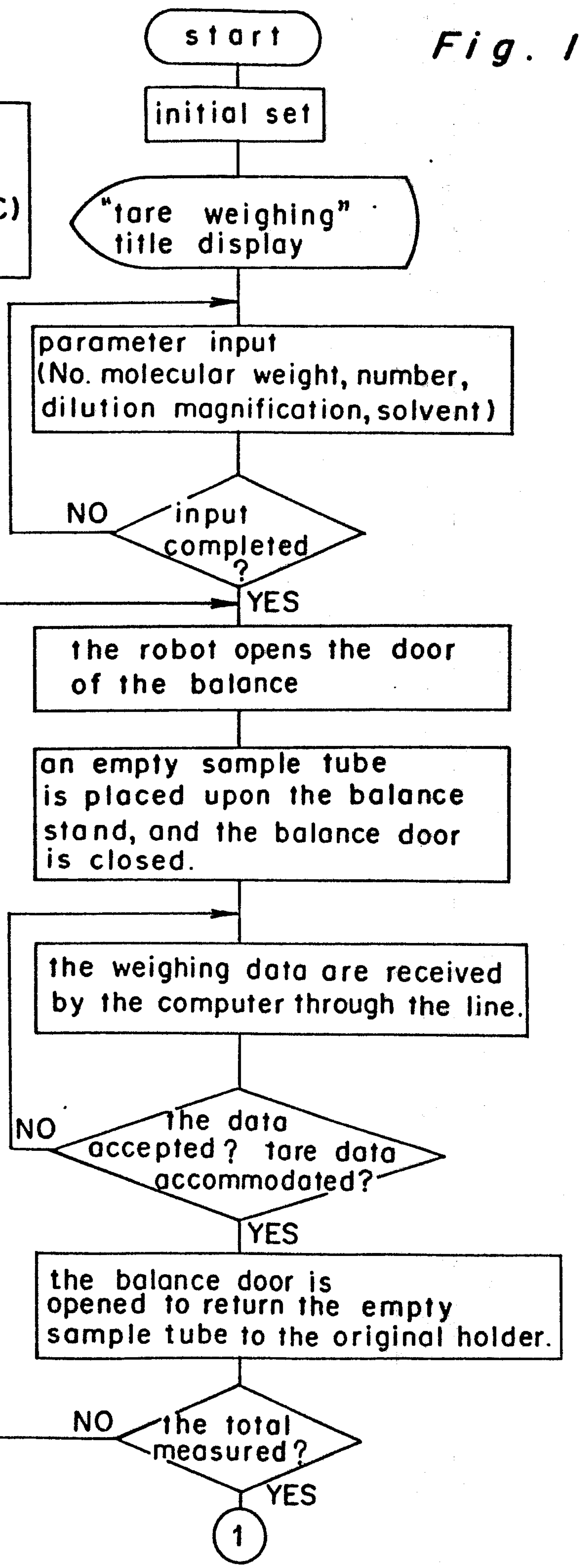

Fig. 10(B)

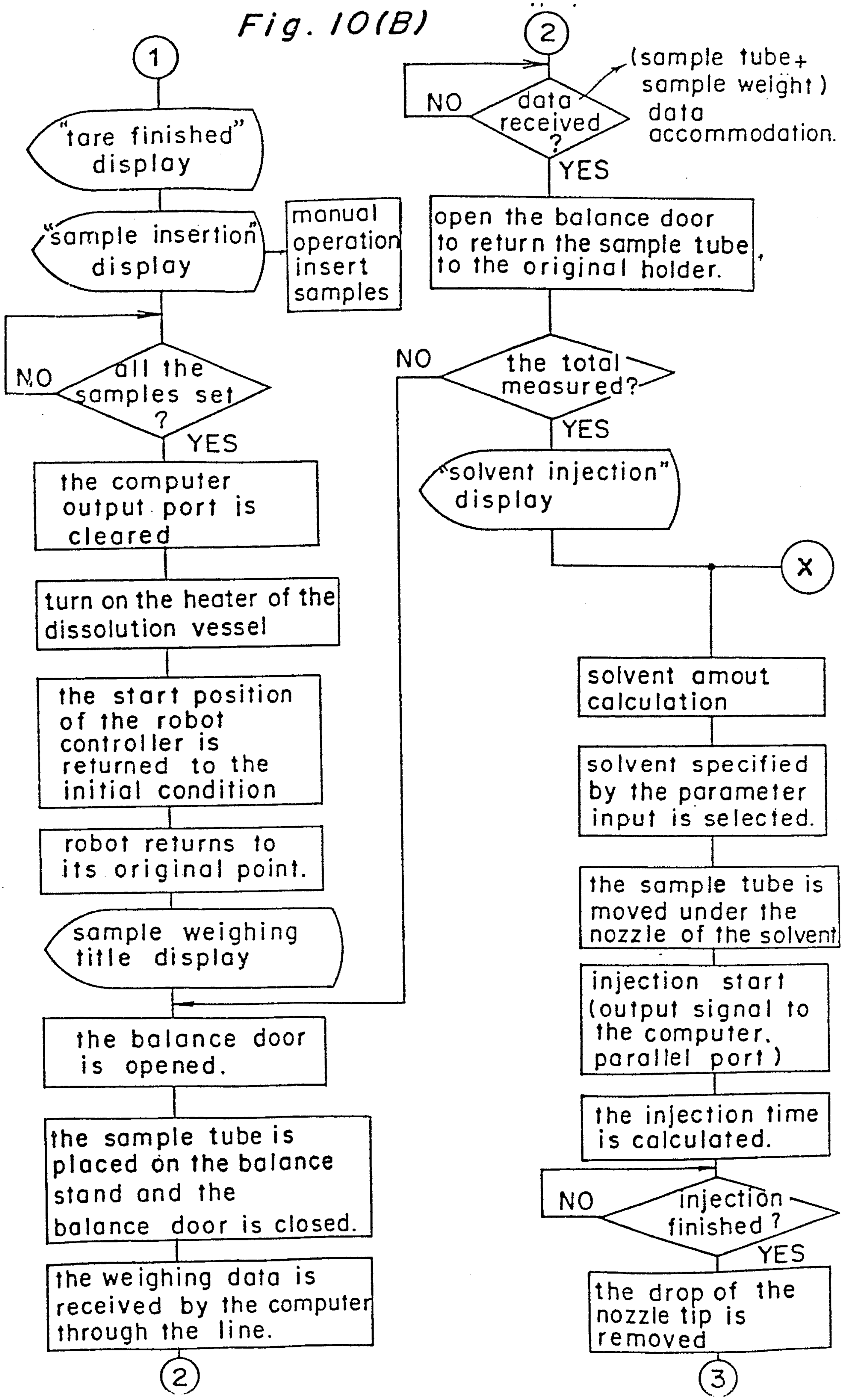
1
"tare finished" display
"sample insertion" display
manual operation insert samples
all the samples set ?
NO
YES
the computer output port is cleared
turn on the heater of the dissolution vessel
the start position of the robot controller is returned to the initial condition
robot returns to its original point.
sample weighing title display
the balance door is opened.
the sample tube is placed on the balance stand and the balance door is closed.
the weighing data is received by the computer through the line.
2
2
data received ?
NO
YES
(sample tube+ sample weight) data accommodation.
open the balance door to return the sample tube to the original holder.
the total measured?
NO
YES
"solvent injection" display
X
solvent amout calculation
solvent specified by the parameter input is selected.
the sample tube is moved under the nozzle of the solvent
injection start (output signal to the computer. parallel port)
the injection time is calculated.
injection finished ?
NO
YES
the drop of the nozzle tip is removed
3

Fig. 10(C)

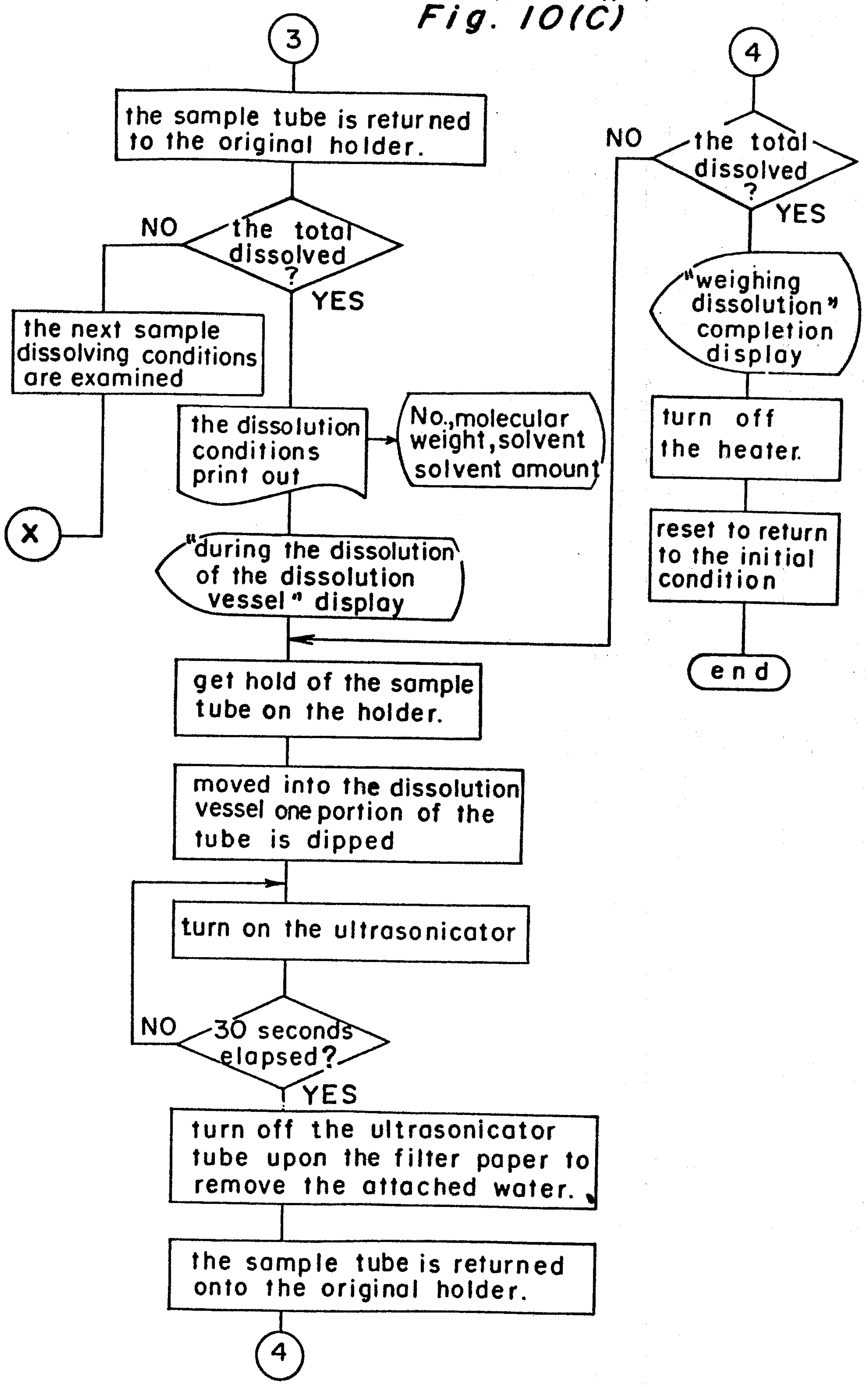
3
the sample tube is returned to the original holder.
the total dissolved ?
NO
YES
the next sample dissolving conditions are examined
X
the dissolution conditions print out
No.,molecular weight,solvent solvent amount
"during the dissolution of the dissolution vessel" display
get hold of the sample tube on the holder.
moved into the dissolution vessel one portion of the tube is dipped
turn on the ultrasonicator
30 seconds elapsed?
NO
YES
turn off the ultrasonicator tube upon the filter paper to remove the attached water.
the sample tube is returned onto the original holder.
4
4
the total dissolved ?
NO
YES
"weighing dissolution" completion display
turn off the heater.
reset to return to the initial condition
end

Fig. 11(A)

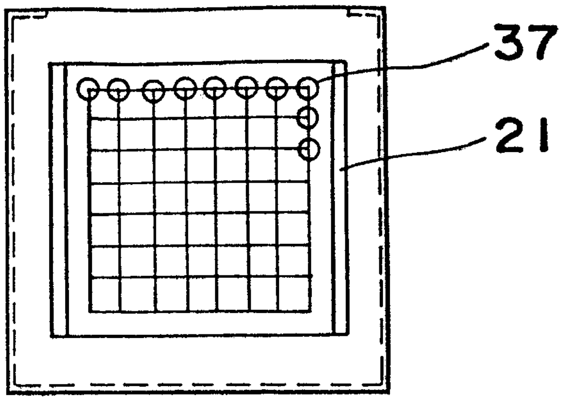


Fig. 11(B)

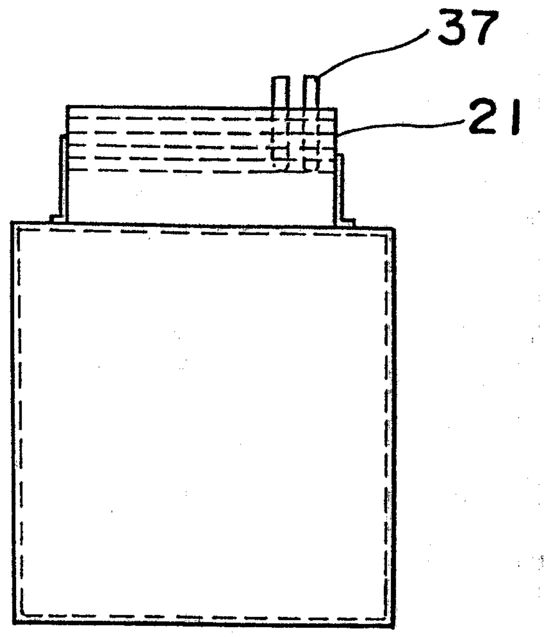


Fig. 12(A)

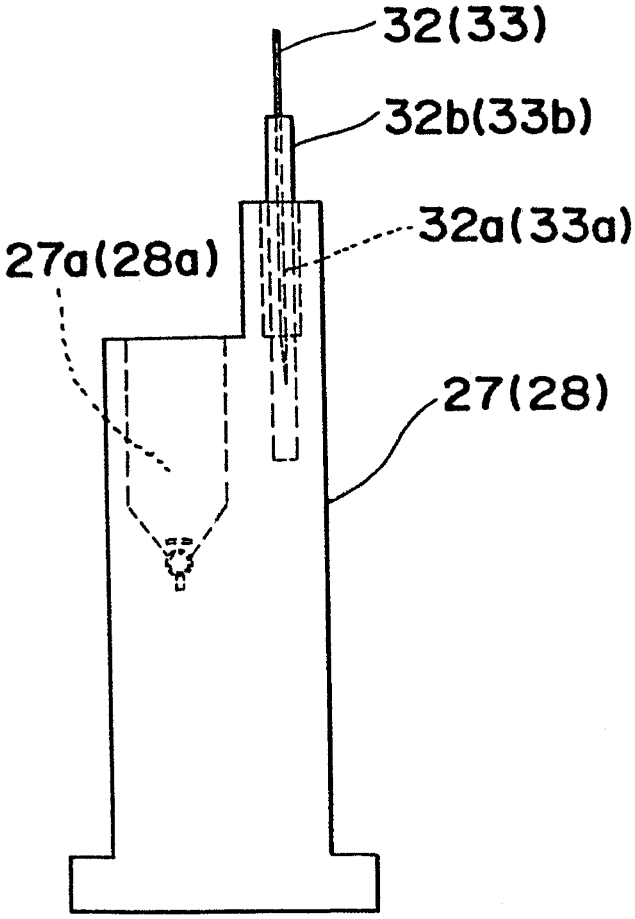


Fig. 12(B)

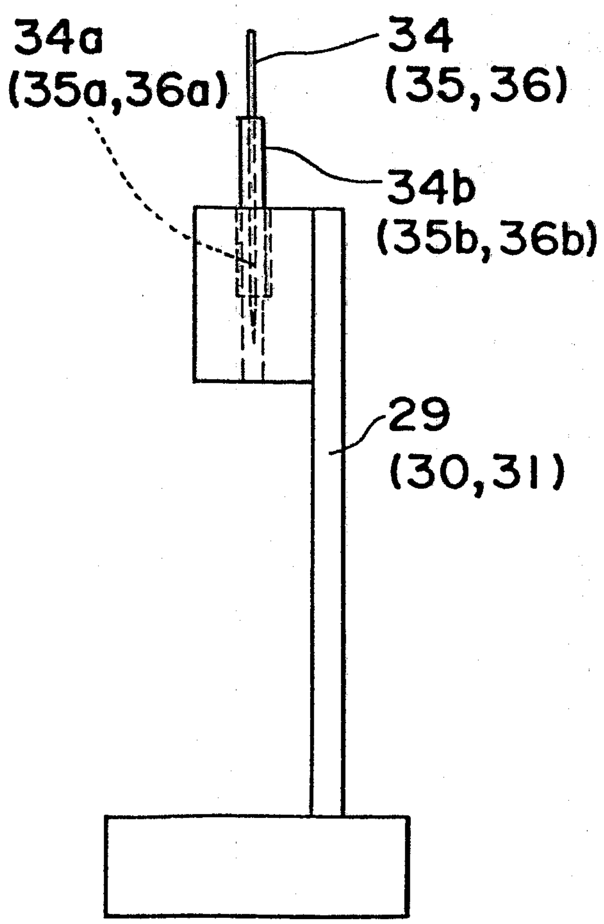

Fig. 13(A)

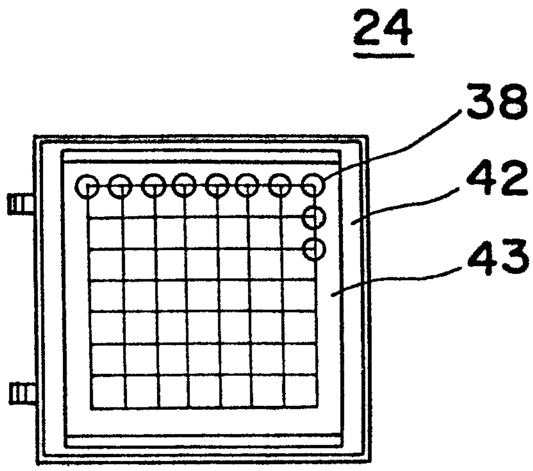


Fig. 13(B)

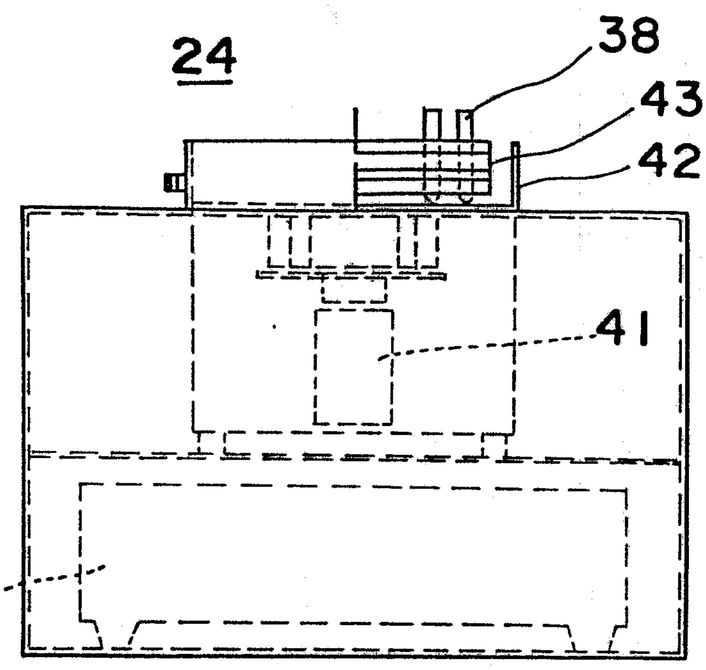


Fig. 14(A)

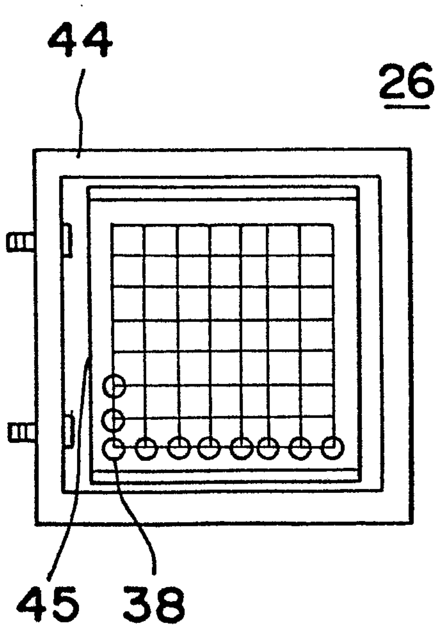


Fig. 14(B)

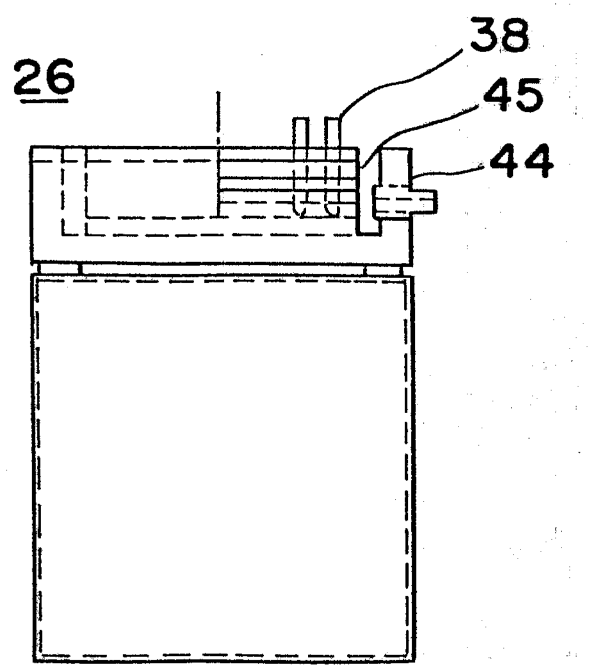

Fig. 15

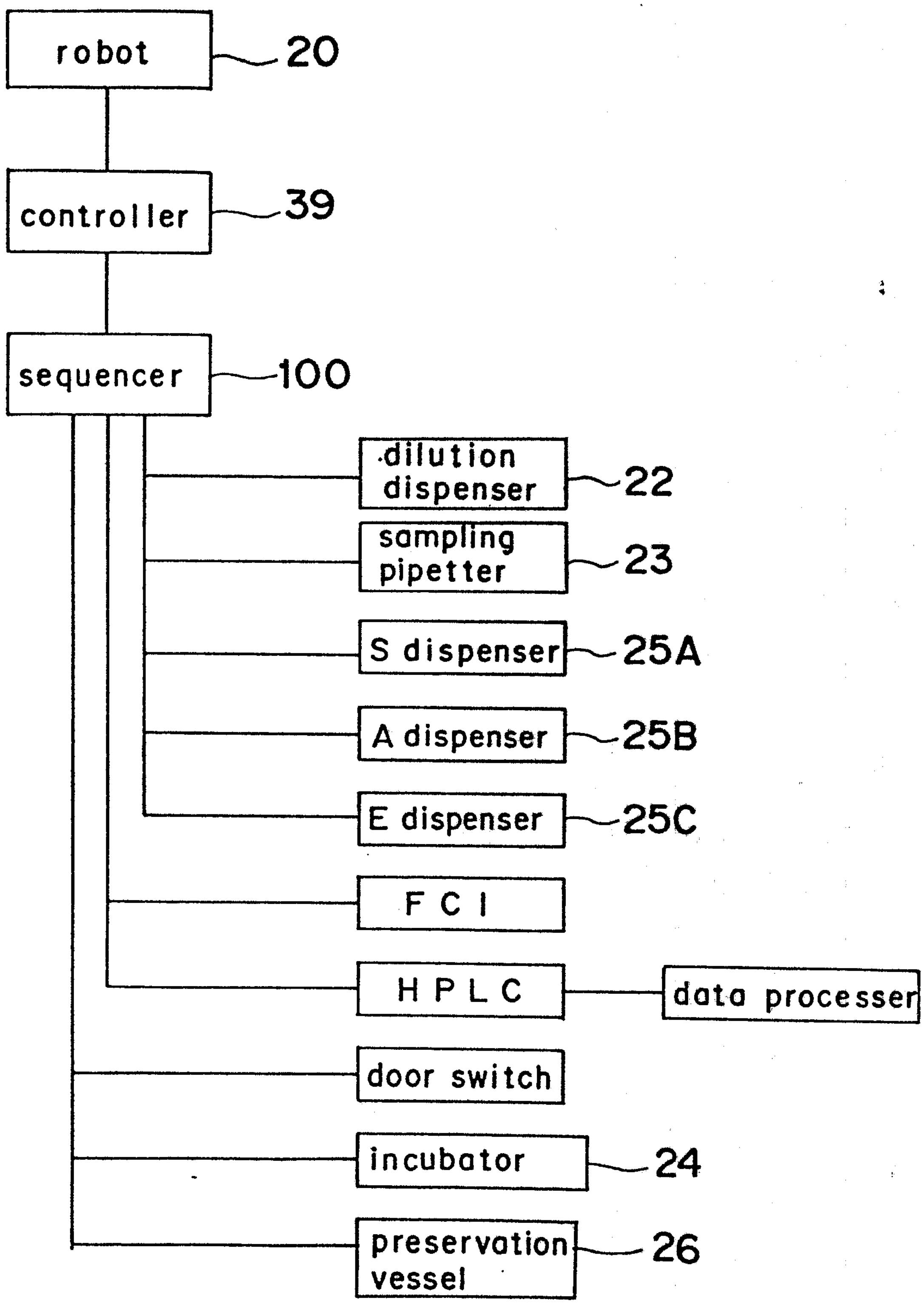
robot
20
controller
39
sequencer
100
dilution dispenser
22
sampling pipetter
23
S dispenser
25A
A dispenser
25B
E dispenser
25C
F C I
H P L C
data processer
door switch
incubator
24
preservation vessel
26

Fig. 16

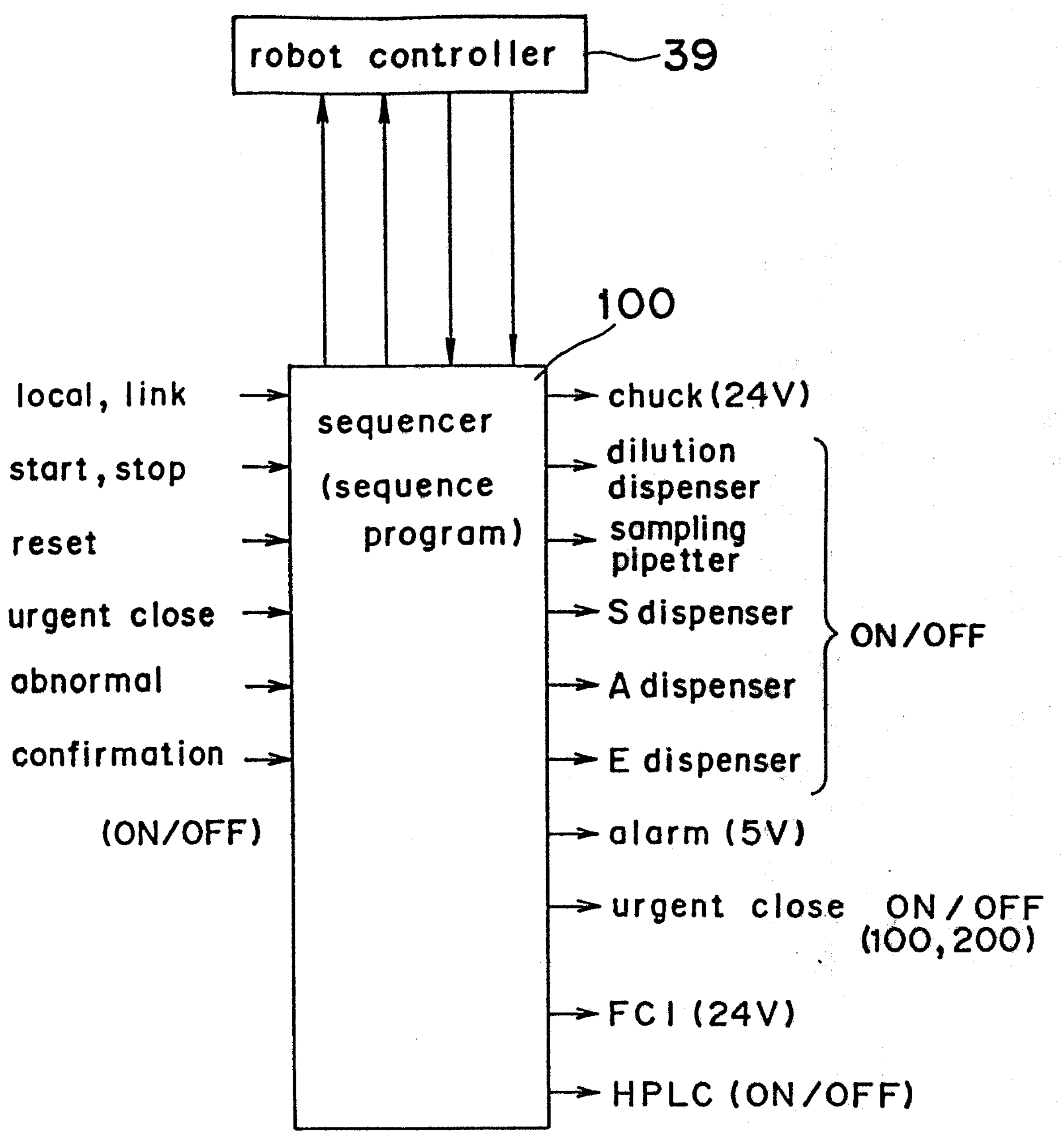
robot controller
39
100
sequencer
(sequence program)
local, link
start, stop
reset
urgent close
abnormal
confirmation
(ON/OFF)
chuck (24V)
dilution dispenser
sampling pipetter
S dispenser
A dispenser
E dispenser
ON/OFF
alarm (5V)
urgent close ON/OFF (100, 200)
FCI (24V)
HPLC (ON/OFF)

Fig. 17

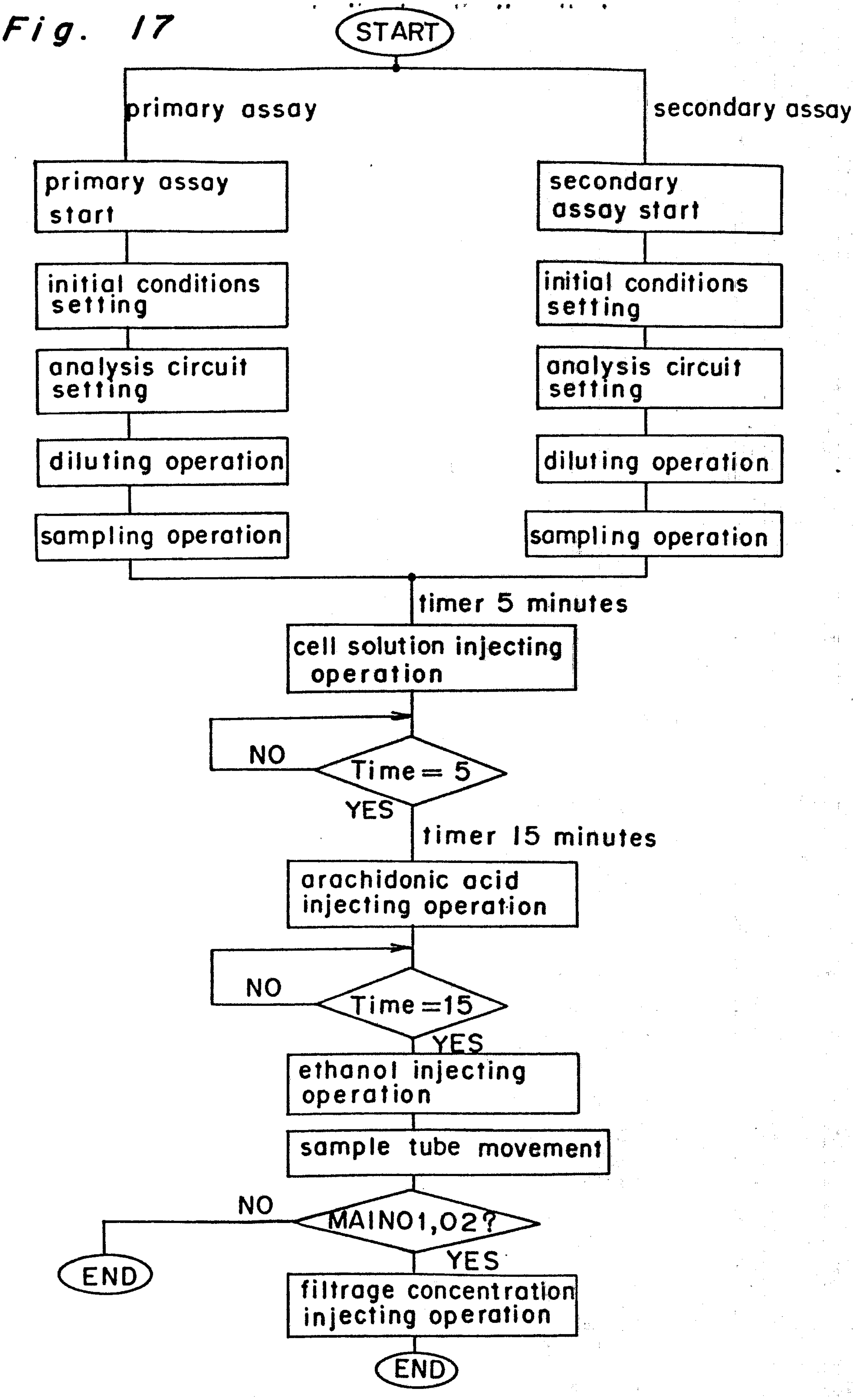
START
primary assay
secondary assay
primary assay start
initial conditions setting
analysis circuit setting
diluting operation
sampling operation
secondary assay start
initial conditions setting
analysis circuit setting
diluting operation
sampling operation
timer 5 minutes
cell solution injecting operation
NO
Time = 5
YES
timer 15 minutes
arachidonic acid injecting operation
NO
Time = 15
YES
ethanol injecting operation
sample tube movement
NO
MAINO1,02?
YES
END
filtrage concentration injecting operation
END

Fig. 18(A)

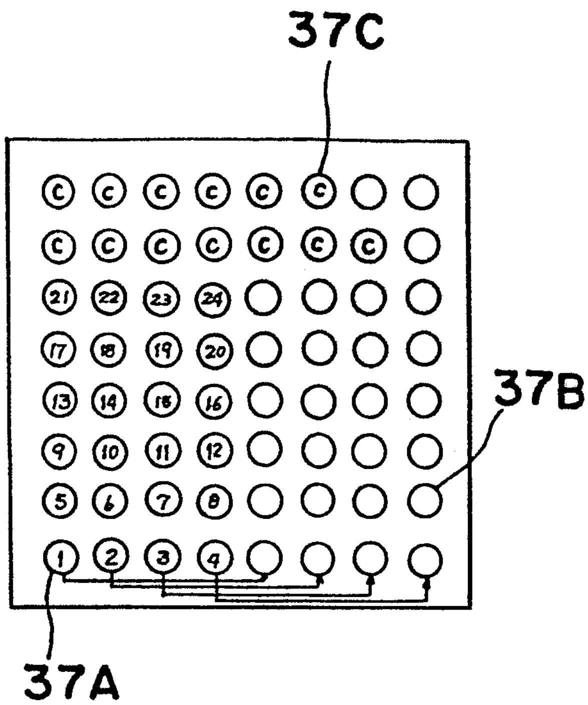


Fig. 18(B)

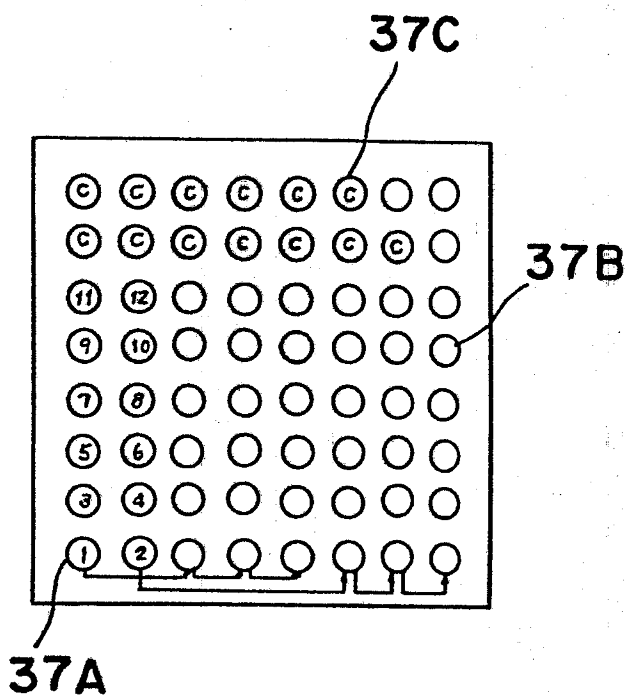


Fig. 20

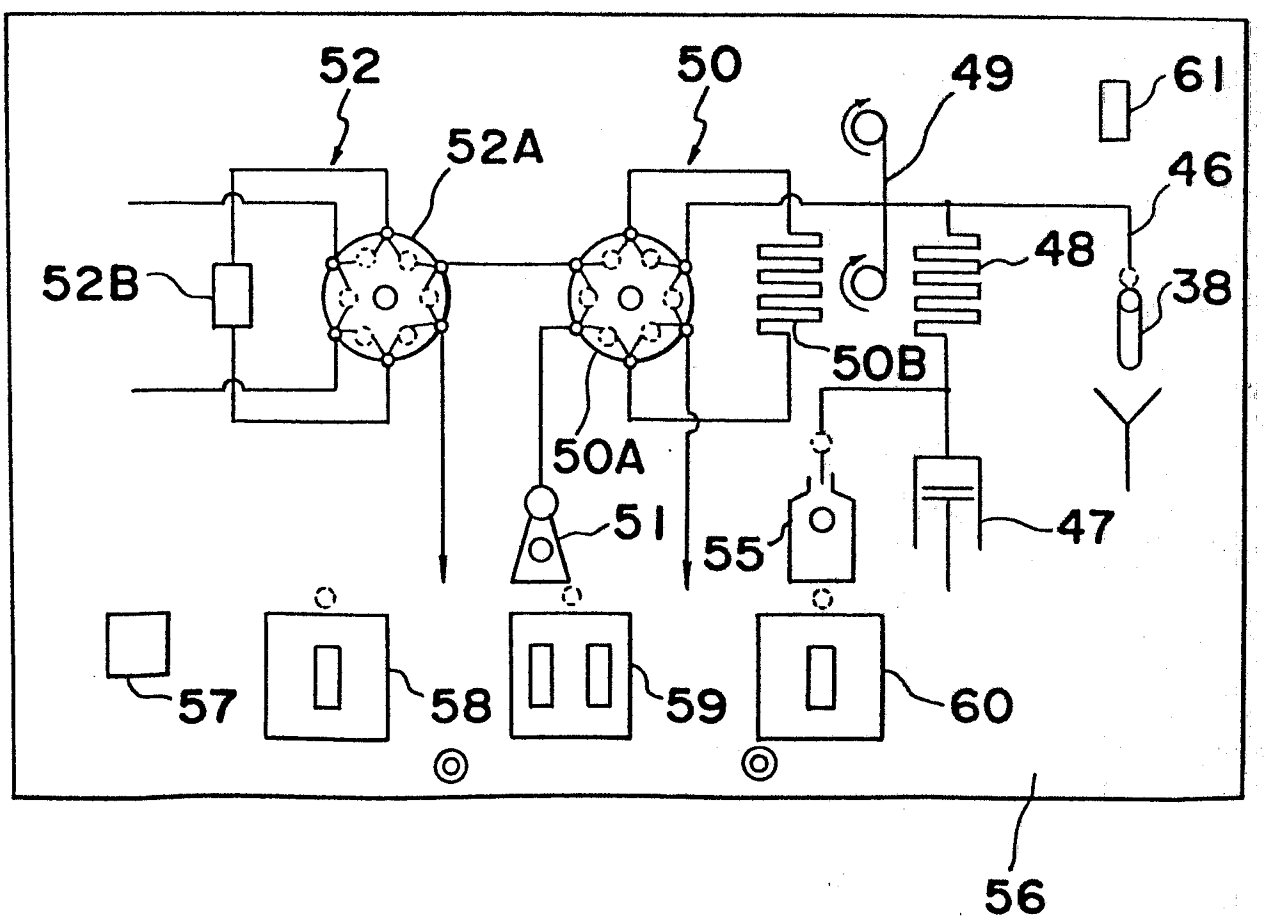

Fig. 19

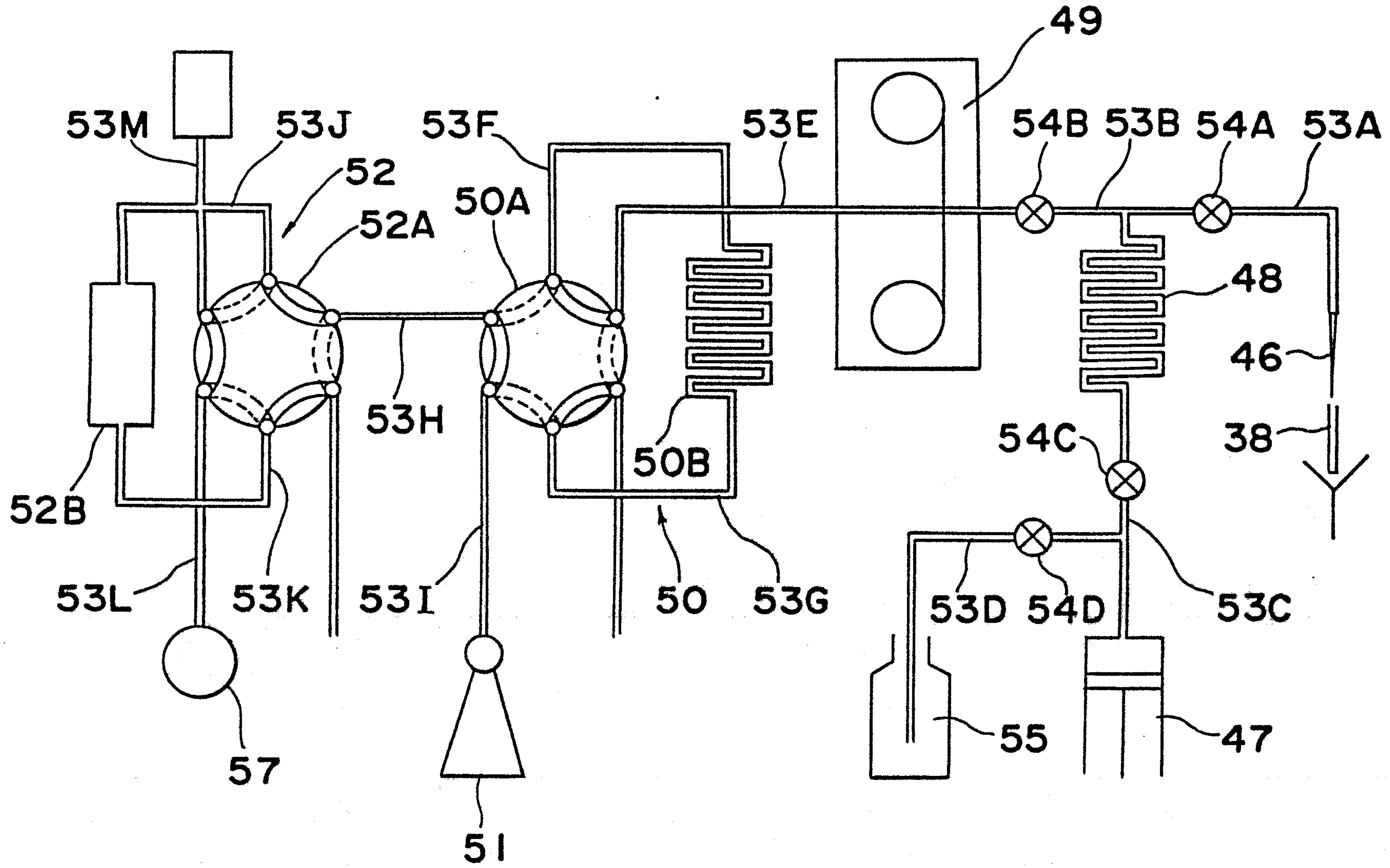
53M
53J
52
52A
50A
53F
53E
49
54B
53B
54A
53A
48
46
38
54C
52B
53H
50B
53L
53K
53I
50
53G
53D
54D
53C
57
51
55
47

Fig. 21

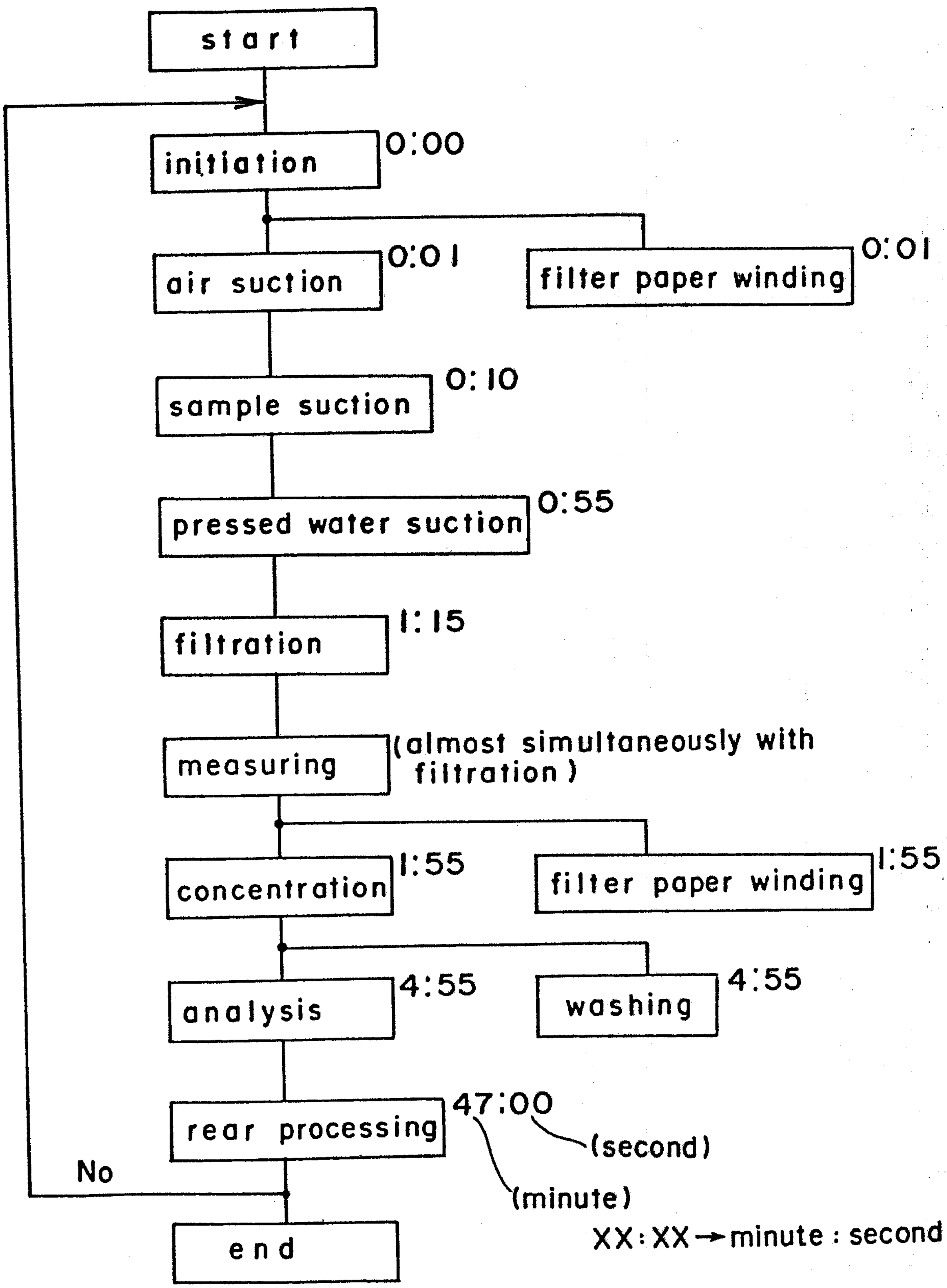
start
initiation
0:00
air suction
0:01
filter paper winding
0:01
sample suction
0:10
pressed water suction
0:55
filtration
1:15
measuring
(almost simultaneously with filtration)
concentration
1:55
filter paper winding
1:55
analysis
4:55
washing
4:55
rear processing
47:00
(second)
(minute)
No
end
XX:XX → minute : second

Fig. 22

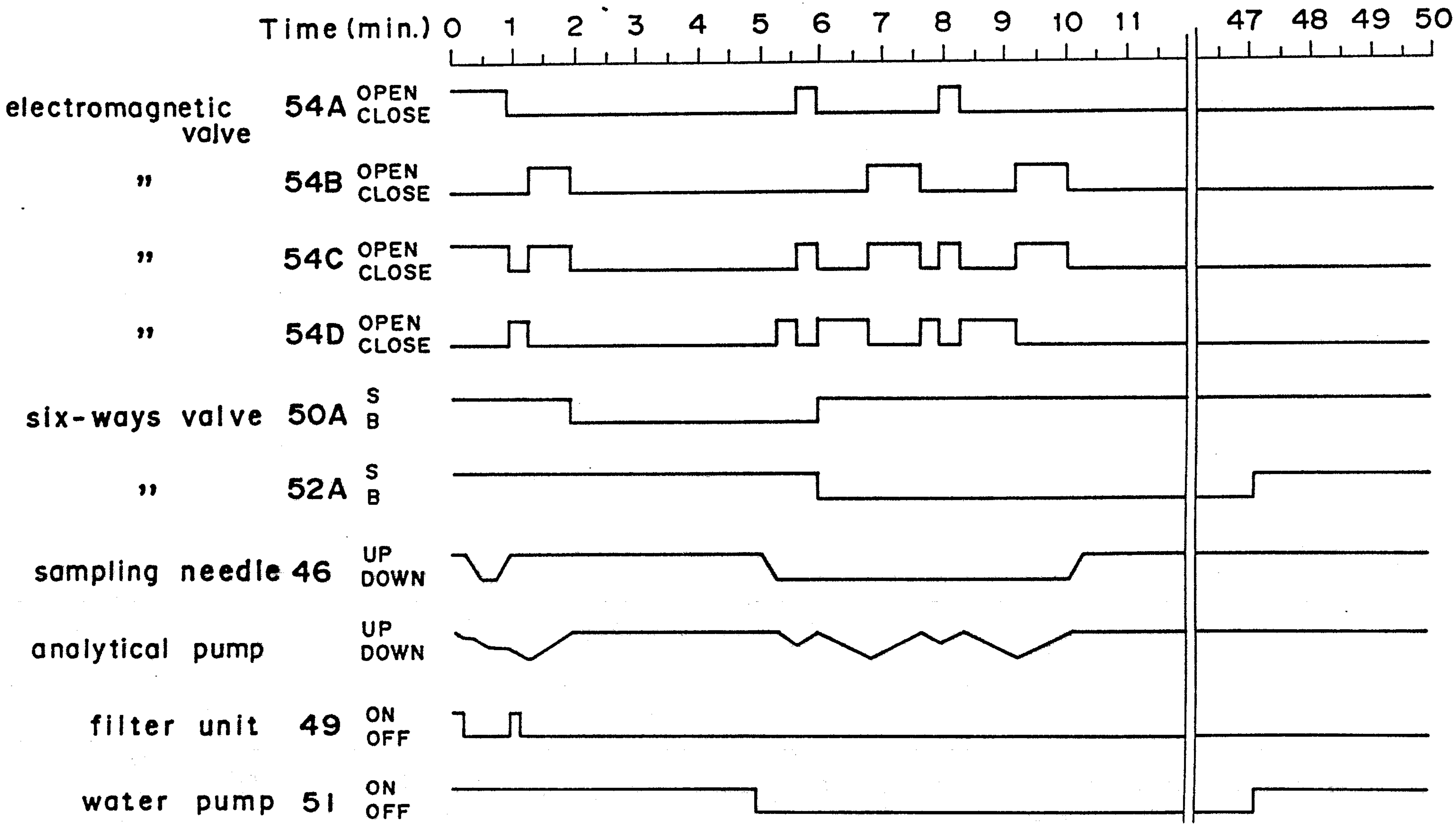
Time (min.)
0
1
2
3
4
5
6
7
8
9
10
11
47
48
49
50
electromagnetic valve
54A
OPEN
CLOSE
"
54B
OPEN
CLOSE
"
54C
OPEN
CLOSE
"
54D
OPEN
CLOSE
six-ways valve
50A
S
B
"
52A
S
B
sampling needle 46
UP
DOWN
analytical pump
UP
DOWN
filter unit
49
ON
OFF
water pump
51
ON
OFF

Fig. 23(A)

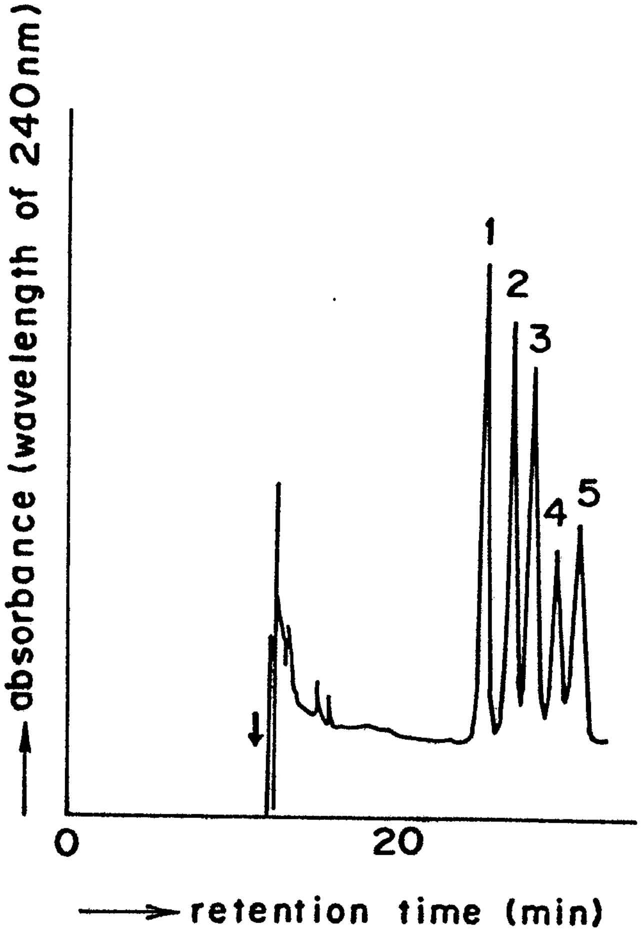


Fig. 23(B)

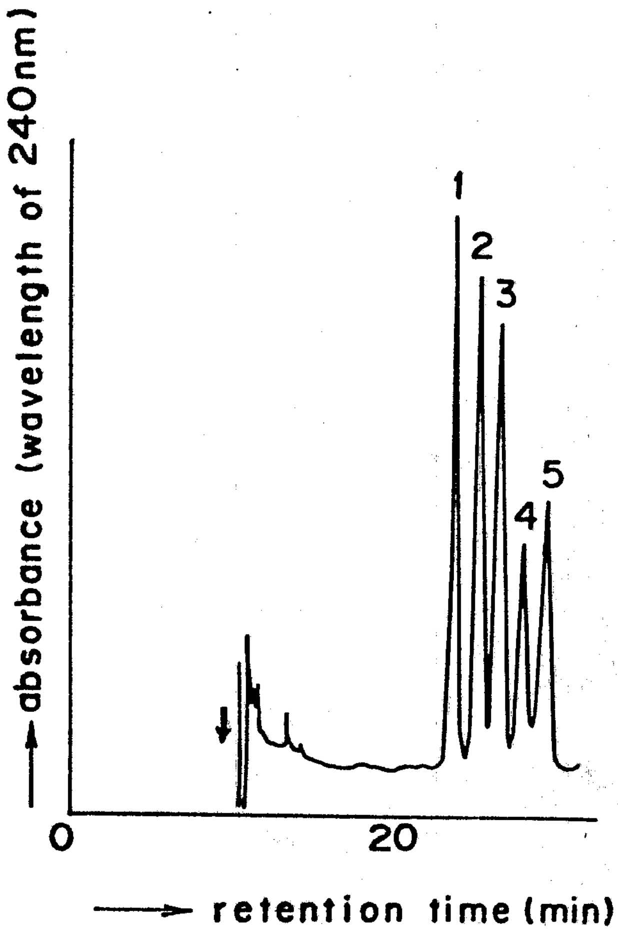


Fig. 23(C)

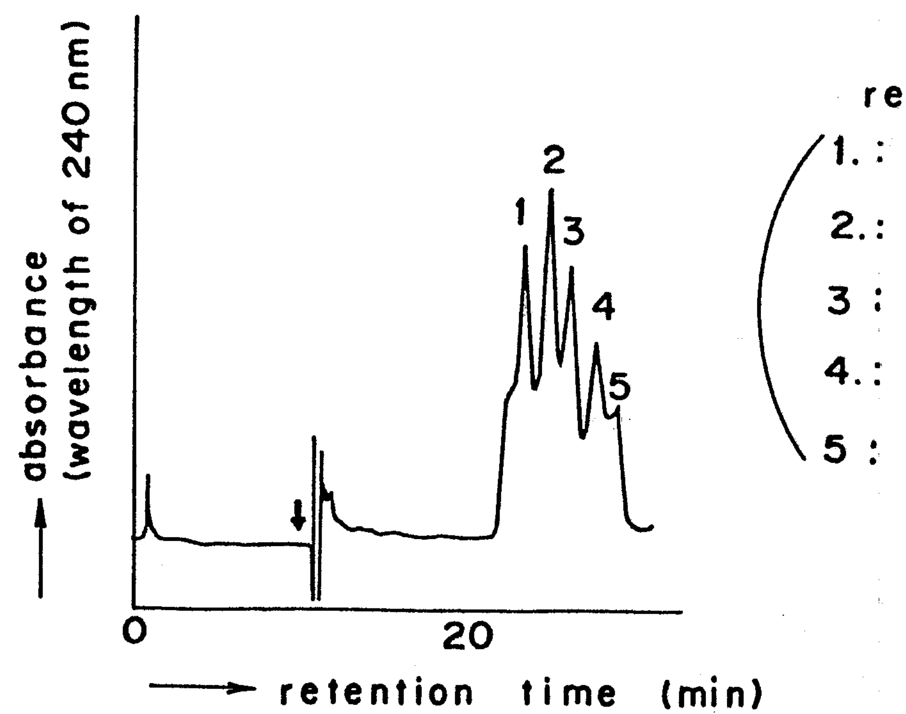


remark

1.: 15-HETE
2.: 11-HETE
3 : 8-,12-HETE
4.: 9-HETE
5 : 5-HETE

22/26

*Fig. 24*

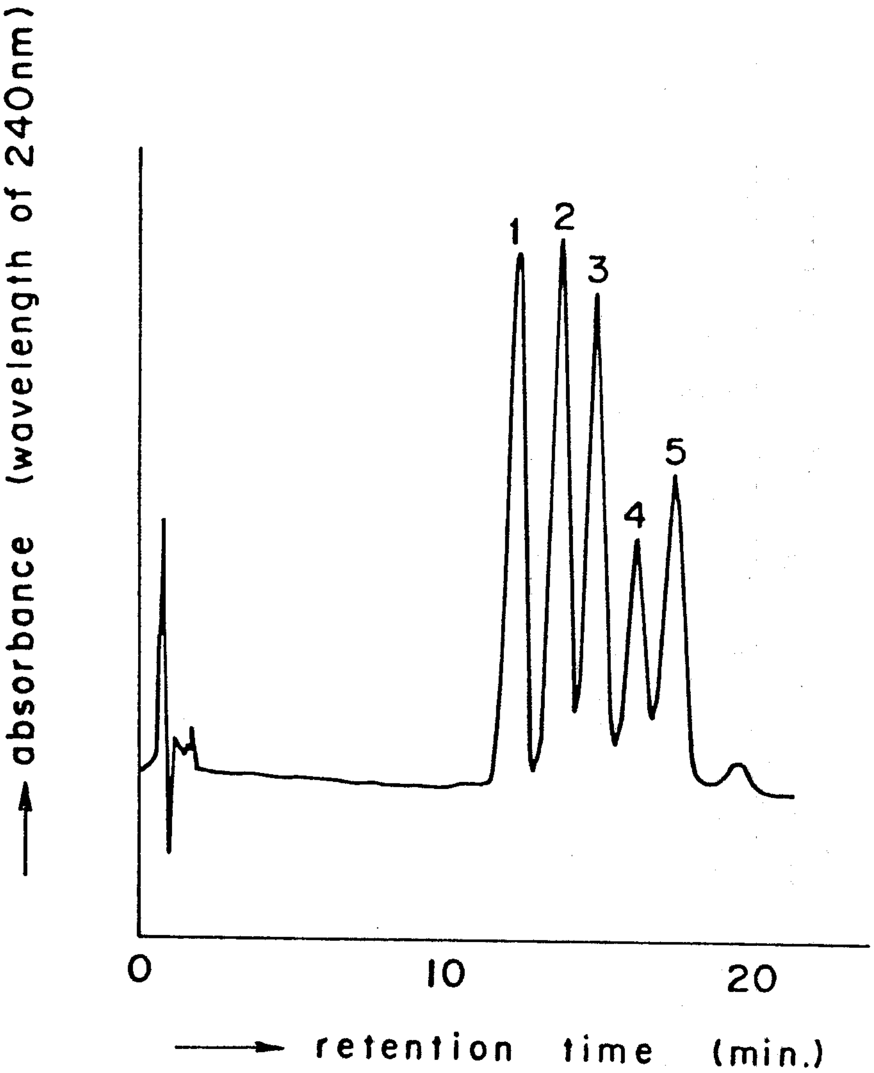


remark

1 : 15-HETE

2 : 11-HETE

3 : 8-,12-HETE

4 : 9-HETE

5 : 5-HETE

*Fig. 25(A)*

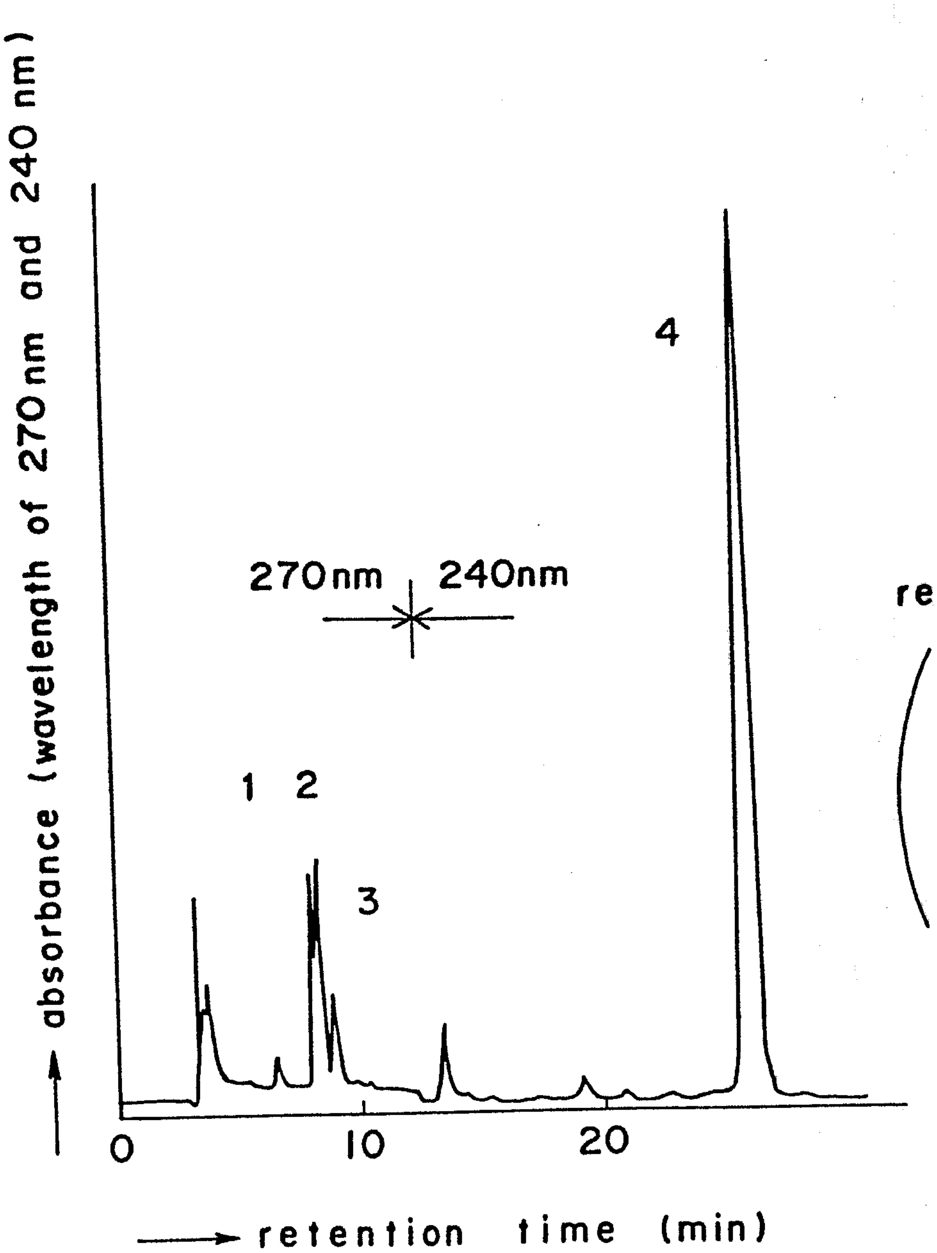


remark

1. 12-epi-$\Delta^6$-trans $LTB_4$
2. $\Delta^6$-trans $LTB_4$
3. $LTB_4$
4. 5-HETE

Fig. 25(B)

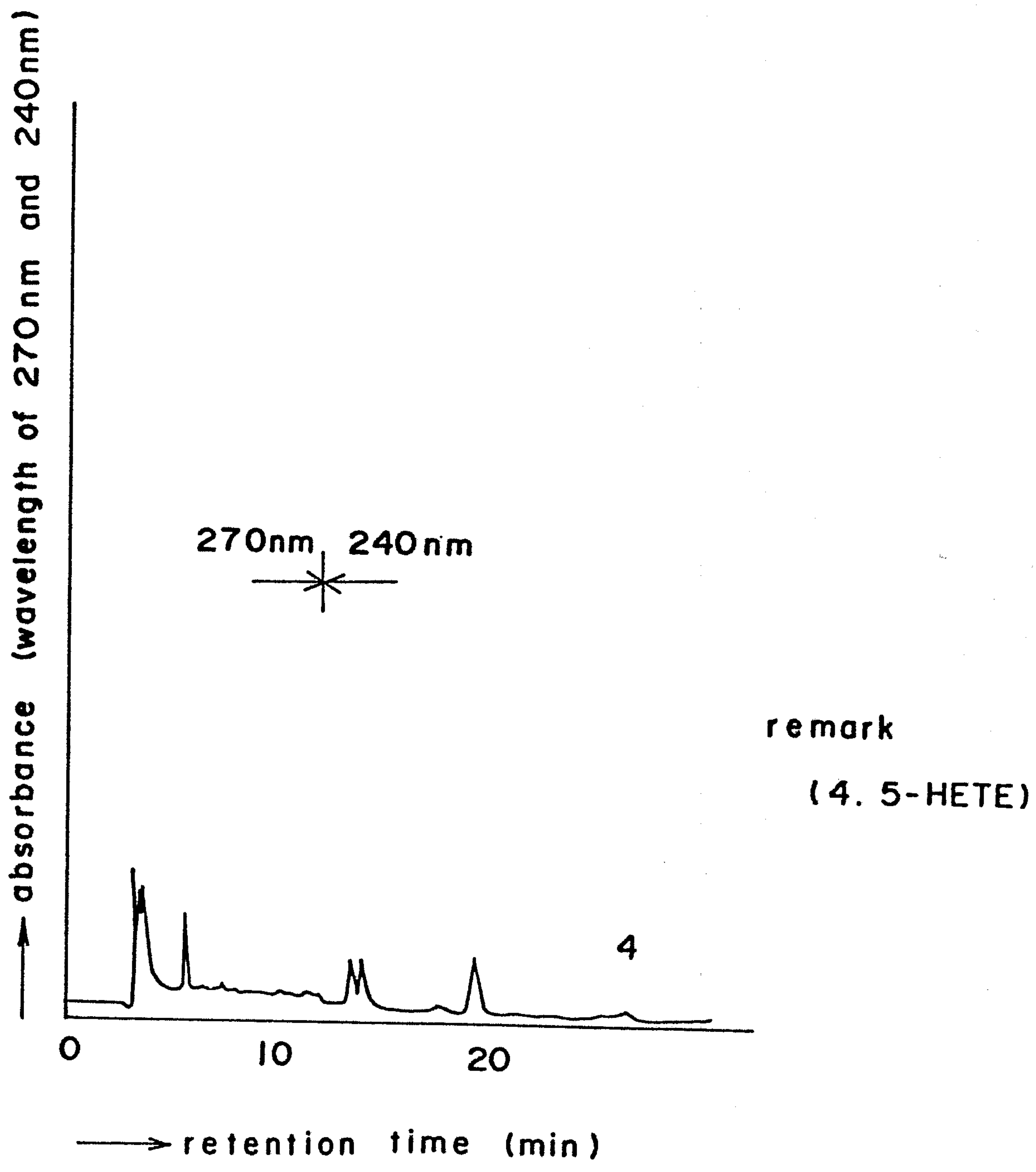
absorbance (wavelength of 270nm and 240nm)
270nm
240nm
remark
(4. 5-HETE)
4
0
10
20
retention time (min)

*Fig. 26*

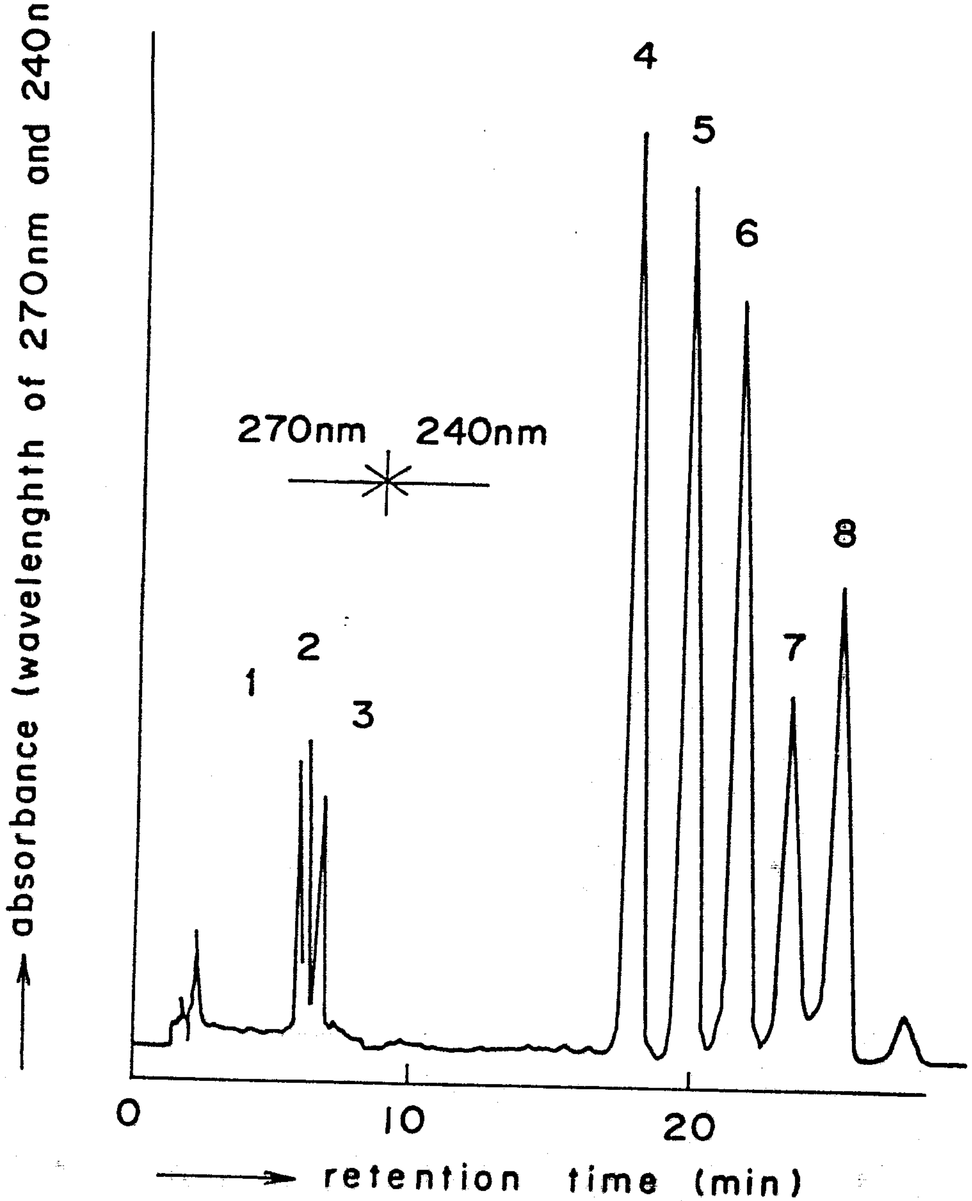


remark

1. 12-epi-$\Delta^6$-trans $LTB_4$
2. $\Delta^6$-trans $LTB_4$
3. $LTB_4$
4. 15-HETE
5. 11-HETE
6. 8-,12-HETE
7. 9-HETE
8. 5-HETE

Fig. 27

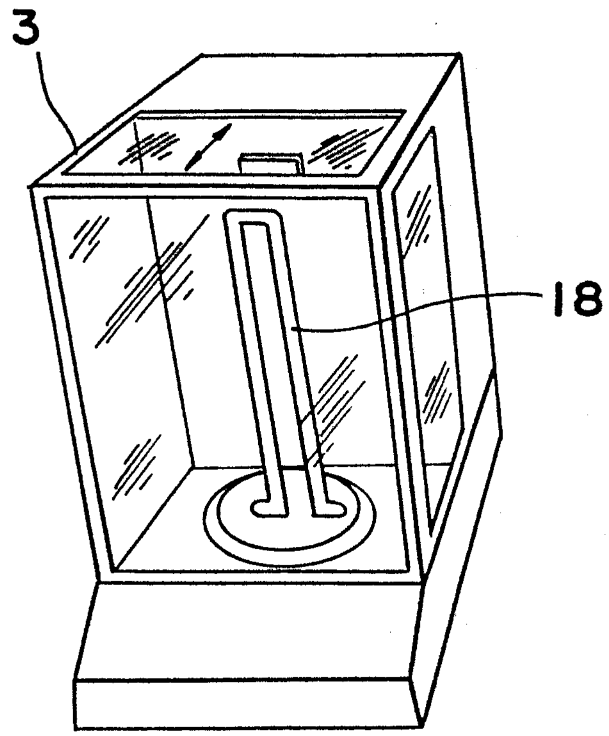


Fig. 28

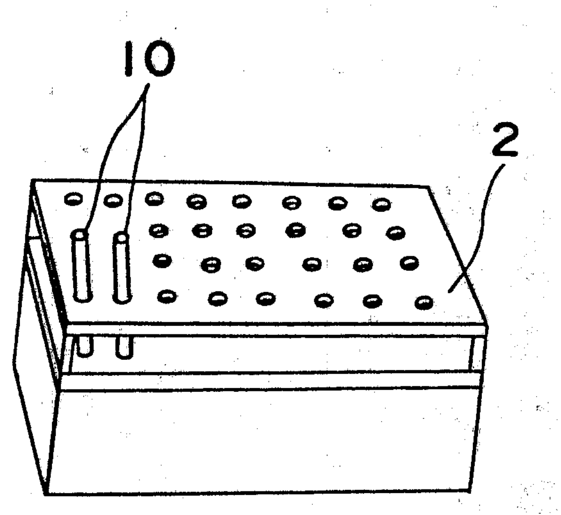


Fig. 29